# EUROPEAN PATENT SPECIFICATION

(11) **EP 0 566 599 B1**
(45) Date of publication and mention of the grant of the patent: **02.04.1997**
(21) Application number: 92901786.1
(22) Date of filing: 08.01.1992
(51) Int. Cl.: G01N 27/62

(54) **A METHOD OF ANALYSIS OF FATTY ACIDS IN TRIACYLGLYCEROLS**
VERFAHREN ZUR ANALYSE VON FETTSÄUREN IN TRIACYLGLYCEROLEN
PROCEDE D'ANALYSE D'ACIDES GRAS DANS DES TRIACYLGLYCEROLS

(30) Priority: 09.01.1991 AU 4146/91
(43) Date of publication of application: 27.10.1993
(73) Proprietor: KALLIO, Heikki Paavo Tapio, SF-21110 Naantali (FI); CURRIE, Graeme James, Pasadena, CA 91196 (US)
(72) Inventor: KALLIO, Heikki Paavo Tapio, SF-21110 Naantali (FI); CURRIE, Graeme James, Pasadena, CA 91196 (US)
(74) Representative: Kummelsten, Per-Arne
(86) International application number: FI9200005
(87) International publication number: WO9212421

(56) References cited:
- EP-A- 0 215 615
- GB-A- 2 187 035
- PATENT ABSTRACTS OF JAPAN, Vol. 12, No. 488, P803, Abstract of JP 63200056, publ 1988-08-18 (SHIMADZU CORP).
- PATENT ABSTRACTS OF JAPAN, Vol. 9, No. 109, P355, Abstract of JP 59231444, publ 1984-12-26 (SHIMAZU SEISAKUSHO K.K.).
- Orbit Search Service, File Analytical Abstracts, Accession Number 49-01D00128, Anal. Chim. Acta, 181, 149-157, 31 March 1986, ROSS, M.M. et al., "Direct fatty acid profiling of complex lipids in intact algae by fast-atom-bombardment mass spectrometry".
- Orbit Search Service, File Analytical Abstracts, Accession Number 49-04D00124, Org. Mass Spectrom., 21 (8) : 485-488, August 1986, BAMBAGIOTTI A.M. et al., "High-energy collisional spectroscopy of AERCOOA-- anions from negative-ion chemical ionization of fatty acid methyl esters".

## Description

This invention relates to a method of analysis and more particularly to a method of analysis of fatty acids in triacylglycerols.

Triacylglycerols or triglycerides are the main components of fats and oils and are comprised of three fatty acids esterified at the stereo-specific number (sn) positions of sn-1, sn-2 and sn-3 of a glycerol molecule. Edible fats and oils such as milk fat, tallow, rape seed oil, fish oil, sunflower oil, peanut oil, evening primrose oil and sea buckthorn seed oil are mixtures of triacylglycerols. Triacylglycerols are also widely used for cosmetic, medical and household purposes in all kinds of fats, oils, emulsions and creams.

Triacylglycerols in fats and oils have many properties which depend upon their fatty acid composition and especially the stereo-specific distribution of the fatty acids on the glycerol moiety. In the present specification the terms sn-1, sn-2 and sn-3 refer to the stereo-specific numbering system defined by the IUPAC nomenclature system. The analysis of fatty acids is a trivial method used everywhere in industry research centres and hospitals. The analysis of the combinations of fatty acids in each triacylglycerol molecule without sn-discrimination is possible in these research units which are equipped with appropriate mass spectrometry facilities. The analysis of the sn-position substituents of the fatty acids in pure triacylglycerols which contain only one species of molecule is possible with combined chromatographic and enzymatic methods but is time consuming and laborious analysis. A proper analysis of sn-position substituents of single molecular species in fats and oils which are typically mixtures of twenty to two hundred different triacylglycerol species is nowadays in practice out of the question because of the extremely high costs and long time needed. For instance it may take several months research effort to analyse the particular composition of the single pure triacylglycerols in an oil such as rape seed oil.

It is important to be able to determine the more detailed structural analysis of the triacylglycerol because their various properties depend upon the fatty acid sn isomers (i.e. enantiomers). The chemical, physical, technical, physiological and medical uses of fats ans oils depend upon their total fatty acid composition, the distribution of the fatty acids at different sn-positions to form individual species of triacylglycerols and the ratio of the individual triacylglycerol species. To be able to determine all of this information for a particular sample would be of great value in the food, cosmetics and medical industries as well as research. The properties which the total composition of fats and oils affect are melting, emulsifying, crystallization, mouth feel, absorption properties, nutrition value and binding capacities.

For a particular example of the use of sn substitution information in the edible fats and oils industry, an integrated part of the refining process is "the mixing and transesterifying unit processes" in which new fat and oil species and varieties are produced. In this process new combination of fatty acids at particular sn-positions are created. At present there is no rapid method which can give sufficient information concerning the flow of the process. The delay in getting this information which at best could be several days (at a rough estimation) is not good enough for a continuous industrial process.

This present invention provides a rapid method of determination of the sn-substituents in a triacylglycerol species.

In another example baby milk substitutes are produced by mixing and processing bovine milk and vegetable oil mixtures. The common and most difficult problem is to know in what quatities and in what combinations palmitic acid (16:0, n-hexadecanoic acid) is located in the formula. This information is necessary because the fat absorption of any infant or adult occurs via absorption of free fatty acids and sn-2 monoacylglycerols. The analysis of palmitic acid at the position sn-2 in the formula with the methods currently available is difficult and for specific molecular species within a mixture almost impossible. It is therefore desirable that triacylglycerol species containing palmitic acid as well as combinations with palmitic acid at the position sn-2 can be analysed reliably and quickly so that industrial process produce the most nutritious or useful forms of baby milk substitutes. Similar problems exist for the manufacture of substitutes for cocoa butter and the stereospecific number information is useful to modify other fats and oils to give the proper mouth feel as well as crystallization and melting properties.

Determination of fatty acids using mass spectrometry is e.g. described in the article of M. Ross et. al. in Anal. Chim. Acta, 181 (1986) 149-157 and in the article of M. Bambagiotti in Org. Mass Spectrom., vol. 21, (1986) 485-488.

It is the object of this invention to provide a method of analysis of triacylglycerols which will give at least some stereo-specific number information in relation to the triacylglycerol molecules. In practice it is only necessary to distinguish between the fatty acid species on the sn-2 position and the fatty acid species on the other two positions sn-1 and sn-3.

It is the object of this invention to provide an analysis method which will give at least this amount of information in relation to a triacylglycerol or mixture of triacylglycerols.

The invention is characterized in what is given in the accompanied patent claims.

In one form therefore although this may not necessarily be the only or broadest form the invention it resides in a method of determination of structural information of a triacylglycerol or a mixture of triacylglycerols comprising the steps of;
generating within a mass spectrometer molecular or quasi-molecular ions which retain the substantial structure of the triacylglycerol or triacylglycerols;
subjecting the ions to a first mass analyser selection process to resolve the ions into first fractions of a particular mass/charge ratio and determining the relative abundance of each first fraction;
selecting in turn each of the first fractions; preferably collisionally activating the ions within each selected first fraction and allowing them or forcing them to decompose into fragment ions;
subjecting the fragment ions to a second mass analyser selection process to resolve the ions into fragments of a particular mass/charge ratio and determining the relative abundance of each fragment ion;
identifying those fragment ions which have mass/charge rations indicating those formulations which provide stereo-specific positional and structural information of the parent molecular or quasi-molecular triacylglycerol ion; and
using the first fraction and fragment ion relative abundance information and the mass/charge ratio information to determine the structure of the triacylglycerol and the proportions of the triacylglycerols of the mixture of triacylglycerols.

It will be seen that the method involves using two sequential mass analysing steps which may be separated in time or space to detect unimolecular decompositions or collisionally induced decompositions of one or more specific ions formed from a triacylglycerol molecule (a triester) or a mixture of molecules. These decompositions may be used to give characteristic information about the triacylglycerol molecule or molecules.

Now looking at the method in more detail the first stage is the production of a molecular of quasi-molecular ion from the triacylglycerol ester being studied. This is most easily performed in one preferred embodiment using ammonia negative ion chemical ionisation, but any ionisation technique that produces a characteristic intense ion that retains the sn-1/3 and sn-2 geometry of the triacylglycerol molecule could be used. The molecular or quasi-molecular ion or ions produced by the chosen ionisation technique are selected by the first quadrupole of a mass analyser. That is, the chosen ion is allowed to pass through the mass analyser and all other non-appropriate ions of different mass/charge ratio are filtered out. The selected ion or ions are then in turn collisionally activated within a second quadrupole of a mass analyser and then the third quadrupole of the mass analyser scans for fragments ions that differentiate the sn-1/3 and sn-2 structre of the parent ion. These fragment ions of interest may be calculated by the general rule [PM-R^{x}CO₂H-X]⁻ where PM is the parent ion selected by the first quadrupole of the mass analyser, R^{x}CO₂H is the sn-1/2/3 fatty acids and X is a constant mass. In the particular case of ammonia negative ion chemical ionisation the fragments of most interest are [PM-R^{x}CO₂H-O]⁻, [PM-R^{x}CO₂H-74]⁻ and [PM-R^{x}CO₂H-100]⁻ with the last one being the most intense group of fragments. The fatty acid at the sn-2 position of the triacylglycerol may then be established since no significant loss of [PM-R²CO₂H-X]⁻ occurs where R²CO₂H is the fatty acid at the sn-2 position.

Although this method has been developed using a triple quadrupole mass spectrometer for the mass analyser the method is applicable to most modern mass spectrometers including double or more stage instruments for instance triple quadrupoles, reverse geometry sector mass spectrometers, triple sector and hybrids, ion traps and ion cyclotron resonance spectrometers.

The first stage of the mass analysis step selects one or more ions derived from the triacylglycerol molecule and filters out unwanted ions. The ion or ions selected are then allowed to undergo unimolecular decomposition or are collisionally activated. A second mass analysis step then scans for one or more fragment ions as discussed above that detail information about the structural identity of the ion selected by the first mass analyser and hence the original triacylglycerol.

An alternative form of mass spectrometer, an ion trap and ion cyclotron resonance mass spectrometer which use sequential mass analysing in time can also be used for the method of the present invention. Ions are generated from a triacylglycerol molecule and then stored within the mass analyser. All ions other than specific parent ions derived from the triacylglycerol molecules are ejected from the mass analyser and the parent ion or ions are allowed to or forced to fragment further. The mass analyser may then scan for fragments of the specific parent ions as discussed above that detail information about the structural identity of the ions selected by the first mass analyser and hence the original triacylglycerol ester.

An alternative form of mass spectrometer which may be used as the mass analyser may be one which uses metastable scanning, for instance linked scans. In this form ions derived from a triacylglycerol molecule or molecules are allowed or forced to decompose in a field free region of a sector mass spectrometer. The magnetic sector and ESA sector are then linked together such that functions of magnetic field strength and ESA field strength remain constant or maintain a constant ratio. The mass spectrometer may then be scanned in a manner that shows ions derived from the triacylglycerol molecule ions and fragmentations associated with those specific ions.

This then generally describes the invention but to assist with understanding of the invention reference will now be made to a number of examples which show the method and results obtainable according to this invention.

The first five examples are examples using known triacylglycerol compounds which can be used to show the method and the rest of the examples describe an example of the application of the method according to this invention to an unknown compound.

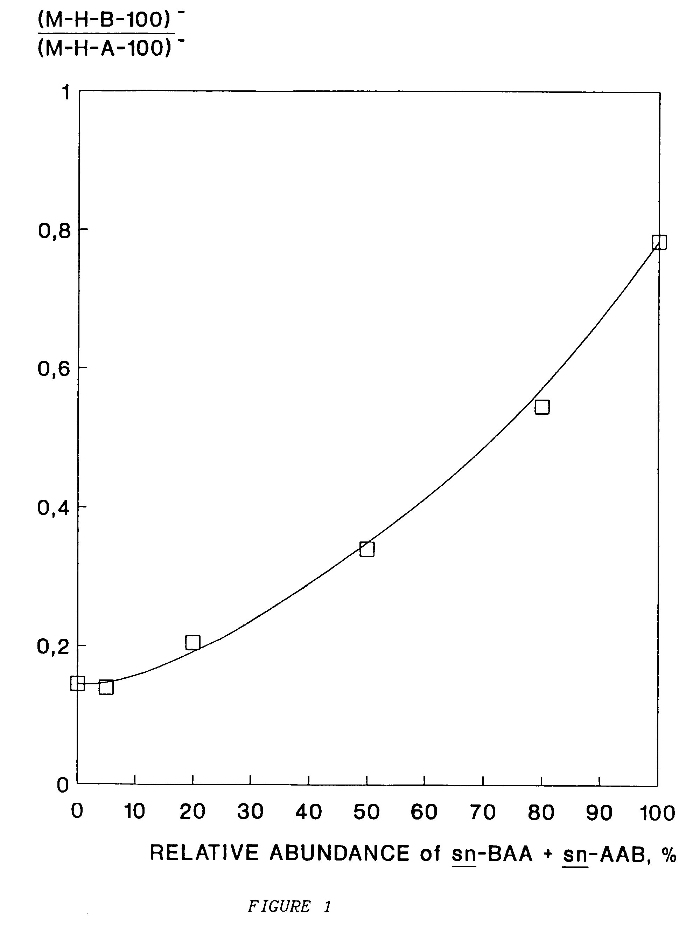

Reference is also made to the following figures in which
Figure 1 shows the dependence of intensity of an [M-H-RCO₂H-100]⁻ ion on the stereospecific position of the corresponding fatty acyl group in a triacylglycerol. Distribution of a fatty acid ('B') between the primary positions (sn-1/3) and the secondary position (sn-2) in a mixture of TAGs containing each of one mole of fatty acid 'B' and two moles of fatty acid(s) 'A'. The graph represents the empirically correlation determined by using mixtures of known ratios of 1,2-di-(cis-9-octadecenoyl)-3-hexadecanoyl-rac-glycerol / 1,3-di-(cis-9-octadecenoyl)-2-hexadecanoylglycerol, 1,2-di-(cis-9-octadecenoyl)-3-octadecanoyl-rac-glycerol / 1,3-di- (cis-9-octadecenoyl)-2-octadecanoylglycerol and 1,2-dioctadecanoyl-3-(cis-9-octadecenoyl)-rac-glycerol / 1,3-dioctadecanoyl-2-(cis-9-octadecenoyl)-glycerol;
Figure 2 shows the ammonia chemical ionization negative ion mass spectrum from purified triacylglycerols of low erucic acid turnip rapeseed oil;
Figure 3 shows the ammonia chemical ionization negative ion mass spectrum from purified triacylglycerols of human milk;
Figure 4 shows the collisional activated daughter spectrum of the [M-H]⁻ parent ion 691.3 of human milk triacylglycerols of 40 acyl carbons and 1 double bond;
Figure 5 shows the collisional activated daughter spectrum of the [M-H]⁻ parent ion 775.2 of human milk triacylglycerols of 46 acyl carbons and 1 double bond;
Figure 6 shows the collisional activated daughter spectrum of the [M-H]⁻ parent ion 855.4 of human milk triacylglycerols of 52 acyl carbons and 3 double bonds;
Figure 7 shows the collisional activated daughter spectrum of the [M-H]⁻ parent ion 857.4 of human milk triacylglycerols of 52 acyl carbons and 2 double bonds;
Figure 8 shows the collisional activated daughter spectrum of the [M-H]⁻ parent ion 873.4 of black currant seed oil of 54 acyl carbons and 8 double bonds. Fatty acid combinations of triacylglycerols are shown in the figure;
Figure 9 shows the collisional activated daughter spectrum of the [M-H]⁻ parent ion 789.4 of human milk of 47 acyl carbons and 1 double bond. Fatty acid combinations of triacylglycerols are shown in figure;
Figure 10 shows the collisional activated daughter spectrum of the [M-H]⁻ parent ion 759.4 of Baltic herring (Clupea harengus membras) flesh triacylglycerols of 45 acyl carbons and 2 double bonds. Fatty acid combinations of triacylglycerols are shown in figure;
Figure 11 shows the collisional activated daughter spectrum of the [M-H]⁻ parent ion 847.3 of Baltic herring (Clupea harengus membras) flesh triacylglycerols of 52 acyl carbons and 7 double bonds. Fatty acid combinations of triacylglycerols are shown in figure.

In the following examples:
14:0 means a fatty acid with 14 carbon atoms and 0 double bonds;
16:2 means a fatty acid with 16 carbon atoms and 2 double bonds;
16:1 means a fatty acid with 16 carbon atoms and 1 double bond;
16:0 means a fatty acid with 16 carbon atoms and 0 double bonds;
18:3 means a fatty acid with 18 carbon atoms and 3 double bonds;
18:2 means a fatty acid with 18 carbon atoms and 2 double bonds;
18:1 means a fatty acid with 18 carbon atoms and 1 double bond;
18:0 means a fatty acid with 18 carbon atoms and 0 double bonds;
20:3 means a fatty acid with 20 carbon atoms and 3 double bonds;
20:1 means a fatty acid with 20 carbon atoms and 1 double bond;
20:0 means a fatty acid with 20 carbon atoms and 0 double bonds.

The actual fatty acids are;
14:0, tetradecanoic acid
16:2, hexadecadienoic acid
16:1, hexadecenoic acid
16:0, hexadecanoic acid
18:3, octadecatrienoic acid
18:2, octadecadienoic acid
18:1, octadecenoic acid
18:0, octadecanoic acid
20:3, eicosatrienoic acid
20:1, eicosenoic acid
20:0, eicosanoic acid

### Example 1.

1-Palmitoyl-2-oleoyl-3-stearoyl-rac-glycerol was introduced into the ion source of a triple quadrupole mass spectrometer using a direct insertion probe, the probe was then heated to a temperature of 310°C. Negative ions were formed by proton abstraction using NH₂ ⁻ as the reagent ion and ammonia as the reagent gas. The first quadrupole was set such that the [M-H]⁻ parent ion was transmitted and all other ions were excluded. The transmitted ion was collisionally activated in the second quadrupole (xenon was used as the collision gas) and the third quadrupole was scanned from a mass to charge ratio (m/z) of 10 to just above the molecular weight of the parent ion [M-H]⁻. Ions were detected using an electron multiplier near the end of the third quadrupole.

The spectrum showed the following product ions
M-H⁻ (4.8) R¹CO₂ ⁻ (93.5) R²CO₂ ⁻ (80.7) R³CO₂ ⁻ (100) [M-H-R¹CO₂H]⁻ (0.9) [M-H-R²CO₂H]⁻ (0.3) [M-H-R³CO₂H]⁻ (1.2) [M-H-R¹CO₂H - 100]⁻ (34.3) [M-H-R²CO₂H -100]⁻ (8.9) [M-H-R³CO₂H -100]⁻ (37.3) [M-H-R¹CO₂H -74]⁻ (1.6) [M-H-R²CO₂H -74]⁻ (0.2) [M-H-R³CO₂H - 74]⁻ (1.6)
where M is the sample molecule being studied, R¹CO₂H is palmitic acid, R²CO₂H is oleic acid and R³CO₂H is stearic acid and the number in ( ) represents the ion current for the ion at that m/z ratio expressed as a precent on the most abundant ion. The abundances of [M-H-R^{1/3}CO₂H-100]⁻ vs [M-H-R²CO₂H-100]⁻ or [M-H-R^{1/3}CO₂H]⁻ vs [M-H-R²CO₂H]⁻ or [M-H-R^{1/3}CO₂H-74]⁻ vs [M-H-R²CO₂H-74]⁻ may be used to determine the proportion of the fatty acid at the sn-2 position.

### Example 2.

1,2-Distearoyl-3-palmitoyl-rac-glycerol was introduced into the ion source of a triple quadrupole mass spectrometer using a direct insertion probe, the probe was then heated to a temparature of 310°C. Negative ions were formed by proton abstraction using NH₂ ⁻ as the reagent ion and ammonia as the reagent gas. The first guadrupole was set such that the [M-H]⁻ parent ion was transmitted and all other ions were excluded. The transmitted ion was collisionally activated in the second quadrupole (xenon was used as the collision gas) and the third quadrupole was scanned from a mass to charge ratio (m/z) of 10 to just above the molecular weight of the parent ion [M-H]⁻. Ions were detected using an electron multiplier near the end of the third quadrupole.

The spectrum showed the following product ions
M-H⁻ (45) R¹CO₂ ⁻ (100) R²CO₂ ⁻ (100) R³CO₂ ⁻ (41.7) [M-H-R¹CO₂H]⁻ (1,0) [M-H-R²CO₂H]⁻ (1.0) [M-H-R³CO₂H]⁻ (0.7) [M-H-R¹CO₂H -100]⁻ (17.8) [M-H-R²CO₂H -100]⁻ (17.8) [M-H-R³CO₂H -100]⁻ (12.3) [M-H-R¹CO₂H -74]⁻ (0.2) [M-H-R²CO₂H -74]⁻ (0.2) [M-H-R³CO₂H - 74]⁻ (0.3)
where M is the sample molecule being studied, R¹CO₂H is stearic acid, R²CO₂H is stearic acid and R³CO₂H is palmitic acid and the number in ( ) represents the ion current for the ion at that m/z ratio expressed as a precent on the most abundant ion. The abundances of [M-H-R^{1/3}CO₂H-100]⁻ vs [M-H-R²CO₂H-100]⁻ or [M-H-R^{1/3}CO₂H]⁻ vs [M-H-R²CO₂H]⁻ or [M-H-R^{1/3}CO₂H-74]⁻ vs [M-H-R²CO₂H-74]⁻ may be used to determine the proportion of the fatty acid at the position sn-2.

### Example 3.

1,3-Dioleyol-2-stearoylglycerol was introduced into the ion source of a triple quadrupole mass spectrometer using a direct insertion probe, the probe was then heated to a temperature of 310°C. Negative ions were formed by proton abstraction using NH₂ ⁻ as the reagent ion and ammonia as the reagent gas. The first quadrupole was set such that the [M-H]⁻ parent ion was transmitted and all other ions were excluded. The transmitted ion was collisionally activated in the second quadrupole (xenon was used as the collision gas) and the third quadrupole was scanned from a mass to charge ratio (m/z) of 10 to just above the molecular weight of the parent ion [M-H]⁻. Ions were detected using an electron multiplier near the end of the third quadrupole.

The spectrum showed the following product ions
M-H⁻ (33) R¹CO₂ ⁻ (100) R²CO₂ ⁻ (58.6) R³CO₂ ⁻ (100) [M-H-R¹CO₂H]⁻ (1,2) [M-H-R²CO₂H]⁻ (0.4) [M-H-R³CO₂H]⁻ (1.2) [M-H-R¹CO₂H -100]⁻ (26) [M-H-R²CO₂H -100]⁻ (3.8) [M-H-R³CO₂H -100]⁻ (26) [M-H-R¹CO₂H -74]⁻ (0.4) [M-H-R²CO₂H -74]⁻ (0.1) [M-H-R³CO₂H - 74]⁻ (0.4)
where M is the sample molecule being studied, R¹CO₂H is oleic acid, R²CO₂H is stearic acid and R³CO₂H is oleic acid and the number in ( ) represents the ion current for the ion at that m/z ratio expressed as a percent of the most abundant ion. The abundances of [M-H-R^{1/3}CO₂H-100]⁻ vs [M-H -R²CO₂H- 100]⁻ or [ M-H -R^{1/3}CO₂H]⁻ vs [M-H-R²CO₂H]⁻ or [M-H-R^{1/3}CO₂H-74]⁻ vs [M-H-R²CO₂H-74]⁻ may be used to determine the proportion of the fatty acid at the position sn-2.

### Example 4.

1,3-Dioleoyl-2-palmitoylglycerol was introduced into the ion source of a triple quadrupole mass spectrometer using a direct insertion probe, the probe was then heated to a temperature of 310°C. Negative ions were formed by proton abstraction using NH₂ ⁻ as the reagent ion and ammonia as the reagent gas. The first quadrupole was set such that the [M-H]⁻ parent ion was transmitted and all other ions were excluded. The transmitted ion was collisionally activated in the second quadrupole (xenon was used as the collision gas) and the third quadrupole was scanned from a mass to charge ratio (m/z) of 10 to just above the molecular weight of the parent ion [M-H]⁻. Ions were detected using an electron multiplier near the end of the third quadruple.

The spectrum showed the following product ions
M-H⁻ (78) R¹CO₂ ⁻ (100) R²CO₂ ⁻ (84.0) R³CO₂ ⁻ (100) [M-H-R¹CO₂H]⁻ (2.3) [N-H-R²CO₂H]⁻ (0.5) [N-H-R³CO₂H]⁻ (2.3) [M-H-R¹CO₂H -100]⁻ (50.2) [M-H-R²CO₂H -100]⁻ (6.5) [M-H-R³CO₂H -100]⁻ (50.2) [M-H-R¹CO₂H -74]⁻ (0.4) [M-H-R²CO₂H -74]⁻ (0.1) [M-H-R³CO₂H - 74]⁻ (0.4)
where M is the sample molecule being studied, R¹CO₂H is oleic acid, R²CO₂H is palmitic acid and R³CO₂H is oleic acid and the number in ( ) represents the ion current for the ion at that m/z ratio expressed as a percent on the most abundant ion. The abundances of [M-H-R^{1/3}CO₂H-100]⁻ vs [M-H-R²CO₂H-100]⁻ or [M-H-R^{1/3}CO₂H]⁻ vs [M-H-R²CO₂H]⁻ or [M-H-R^{1/3}CO₂H-74]⁻ vs [M-H-R²CO₂H-74]⁻ may be used to determine the proportion of the fatty acid at the position sn-2.

### Example 5.

1,2-Dioleoyl-3-palmitoyl-rac-glycerol was introduced into the ion source of a triple quadrupole mass spectrometer using a direct insertion probe, the probe was then heated to a temperature of 310°C. Negative ions were formed by proton abstraction using NH₂ ⁻ as the reagent ion and ammonia as the reagent gas. The first quadrupole was set such that the [M-H]⁻ parent ion was transmitted and all other ions were excluded. The transmitted ion was collisionally activated in the second quadrupole (xenon was used as the collision gas) and the third quadrupole was scanned from a mass to charge ratio (m/z) of 10 to just above the molecular weight of the parent ion [M-H]⁻. Ions were detected using an electron multiplier near the end of the third quadrupole.

The spectrum showed the following product ions
M-H⁻ (16.4) R¹CO₂ ⁻ (100) R²CO₂ ⁻ (100) R³CO₂ ⁻ (52.5) [M-H-R¹CO₂H]⁻ (0.6) [M-H-R²CO₂H]⁻ (0.6) [N-H-R³CO₂H]⁻ (0.7) [M-H-R¹CO₂H -100]⁻ (20.3) [M-H-R²CO₂H -100]⁻ (20.3) [M-H-R³CO₂H -100]⁻ (15.9) [M-H-R¹CO₂H -74]⁻ (0.7) [M-H-R²CO₂H -74]⁻ (0.7) [M-H-R³CO₂H - 74]⁻ (0.4)
where M is the sample molecule being studied, R¹CO₂H is oleic acid, R²CO₂H is oleaic acid and R³CO₂H is palmitic acid and the number in ( ) represents the ion current for the ion at that m/z ratio expressed as a precent on the most abundant ion. The abundances of [M-H-R^{1/3}CO₂H-100]⁻ vs [M-H-R²CO₂H-100]⁻ or [M-H-R^{1/3}CO₂H]⁻ vs [M-H-R²CO₂H]⁻ or [M-H-R^{1/3}CO₂H-74]⁻ vs [M-H-R²CO₂H-74]⁻ may be used to determine the proportion of the fatty acid at the position sn-2.

Table I summarizes the partial ammonia negative ion chemical ionization collisional activated mass spectra of some known reference triacylglycerols.

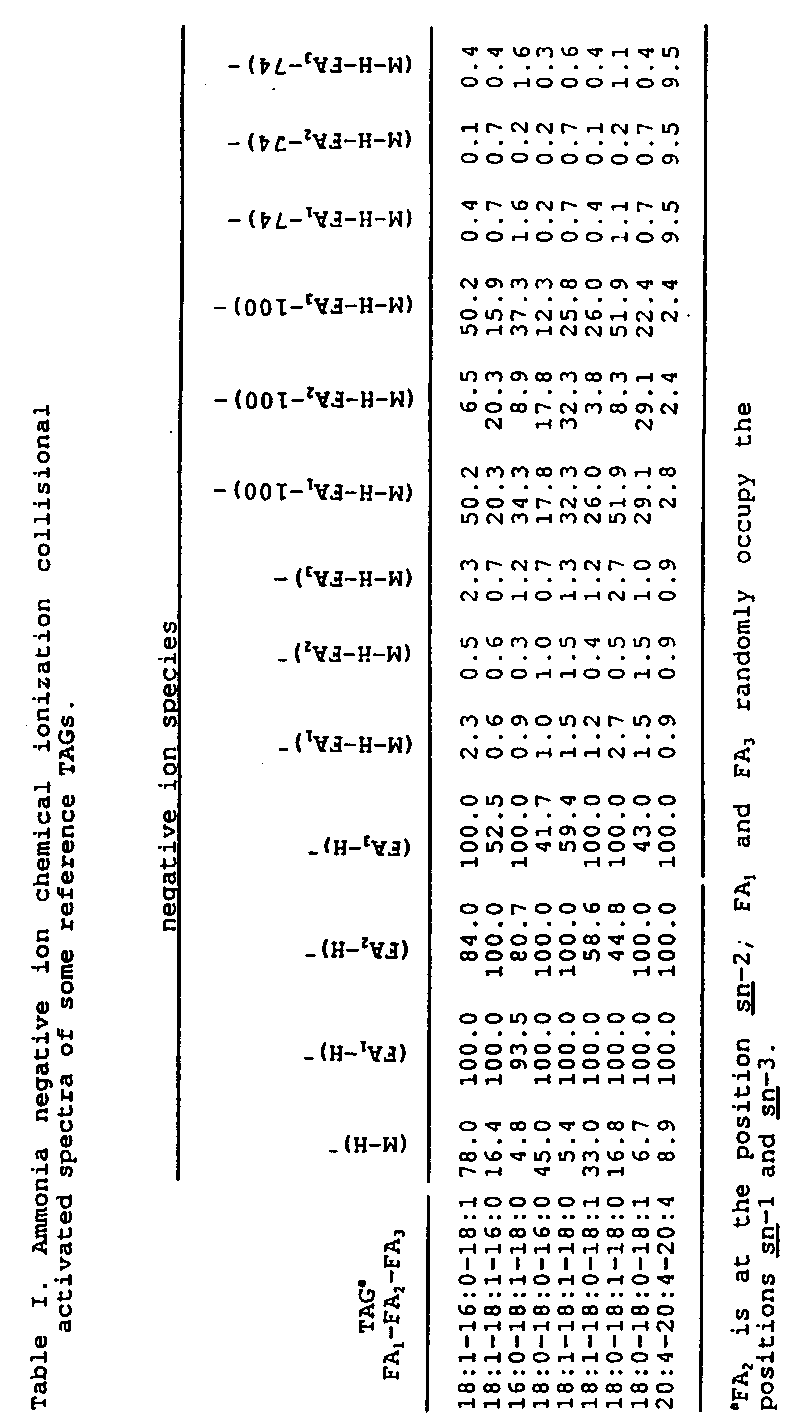

Table I and Examples 1 through 5 show, that in the TAG (TriAcylGlycerol) system R¹CO₂H-R²CO₂H-R³CO₂H the ratio of the intensities [M-H-R²CO₂H-100]⁻/ ([M-H-R¹CO₂H-100]⁻ + [M-H-R³CO₂H-100]⁻⁾ appeared to be 0.145±0.016, whereas the ratio of the intensities [M-H-R¹CO₂H-100]⁻/ ([M-H-R²CO₂H-100]⁻ + [M-H-R³CO₂H-100]⁻) appeared to be 0.784±0.015. When analyzing mixtures of known ratios of known reference compounds of Table I and examples 1 through 5, a calibration curve shown in Figure 1 was obtained. Figure 1 was applied in the Examples 6 through 11 when determining stereospecific positions of the fatty acyl groups in TAGs.

The designation R¹CO₂H-R²CO₂H-R³CO₂H means a triacylglycerol having R¹CO₂H fatty acid at the sn-1 or sn-3 position, a R²CO₂H fatty acid at the sn-2 position and a R³CO₂H fatty acid at the sn-1 or sn-3 position. The TAG is thus either sn-R¹CO₂H-R²CO₂H-R³CO₂H or sn-R³CO₂H-R²CO₂H-R¹CO₂H.

By using known, pure reference triacylglycerols such as TAGs shown in Table I, molar correction factors were empirically determined for some fatty acids by using the intensities of the corresponding RCO₂ ⁻ ions of the CA spectra of the TAGs shown in Table II:
TABLE II. Molar correction factors of RCO₂ ⁻ ions of the collisional activated spectra of triacylglycerols for some fatty acids

| FATTY ACID | CORRECTION FACTOR |
|---|---|
| 14:0 | 1.3 |
| 16:0 | 1.1 |
| 16:1 | 1.5 |
| 16:2 | 1.7 |
| 18:0 | 1.0 |
| 18:1 | 1.3 |
| 18:2 | 1.6 |
| 18:3 | 1.9 |

The mass spectrometric conditions may affect the correction factors and thus the analytical conditions have to be kept constant.

### Example 6. Determination of the fatty acid combinations in triacylglycerols

Analysis of fatty acid combinations in triacylglycerols and the proportions of these combinations in complex TAG mixtures such as turnip rapeseed oil, borage oil, or human milk is extremely difficult and labourous using commonly known methods. By applying the method invented in this application, analysis of TAG mixtures of samples of turnip rapeseed oil, borage oil and human milk were examined and resolved.

Intensities of the RCO₂ ⁻ ions in the collisional activated (CA) daughter spectra of [M-H]⁻ parent ions were multiplied by the molar correction factors determined of the reference TAGs (Table II). The corrected intensities of RCO₂ ⁻ ions were then used to calculate the ratios of the fatty acid combinations within each MW species. The parent ions, [M-H]⁻, contained information of the acyl carbon numbers and of the number of double bonds in TAGs.

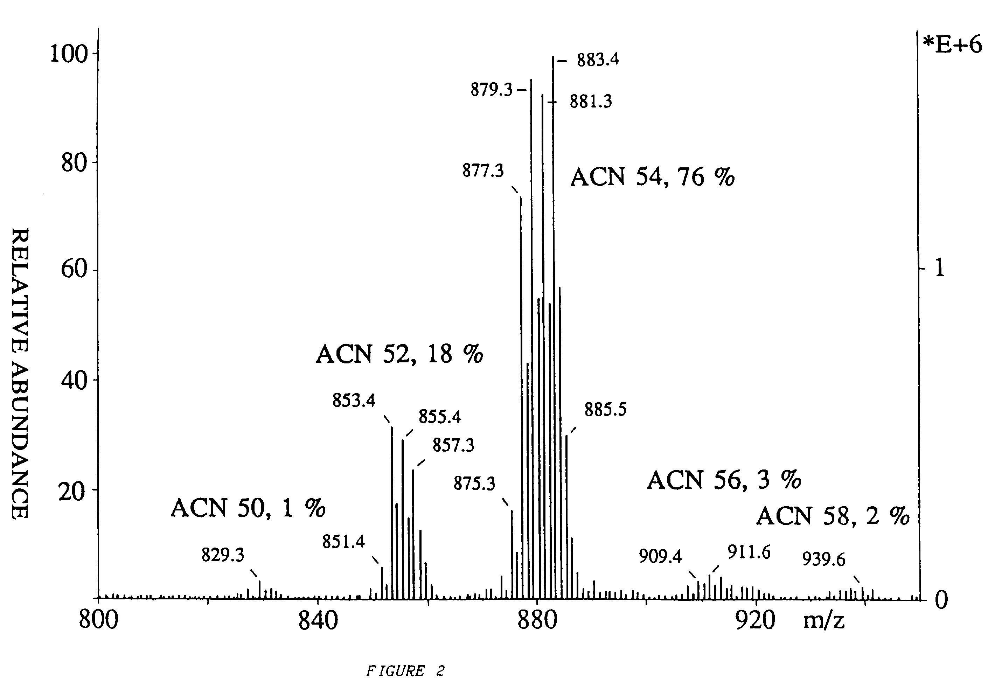

6.1. Turnip rapeseed oil was analyzed by ammonia negative ion chemical ionization mass spectrometry (NICI-MS) and a mass spectrum is reproduced in Figure 2. Proportions of the TAGs of different molecular weights were determined from the NICI mass spectra. The results were corrected by subtracting the ¹³C isotope ions and the results are shown in Table III.

Table III has been divided into four pages as the table contains so much information that cannot be expressed clearly on one page. So Tables III A, B, C and D are read in the following pattern:
A B
C D

Table III. Ammonia chemical ionization tandem mass spectra results of purified triacylglycerols of turnip rapeseed oil obtained by supercritical fluid extraction.

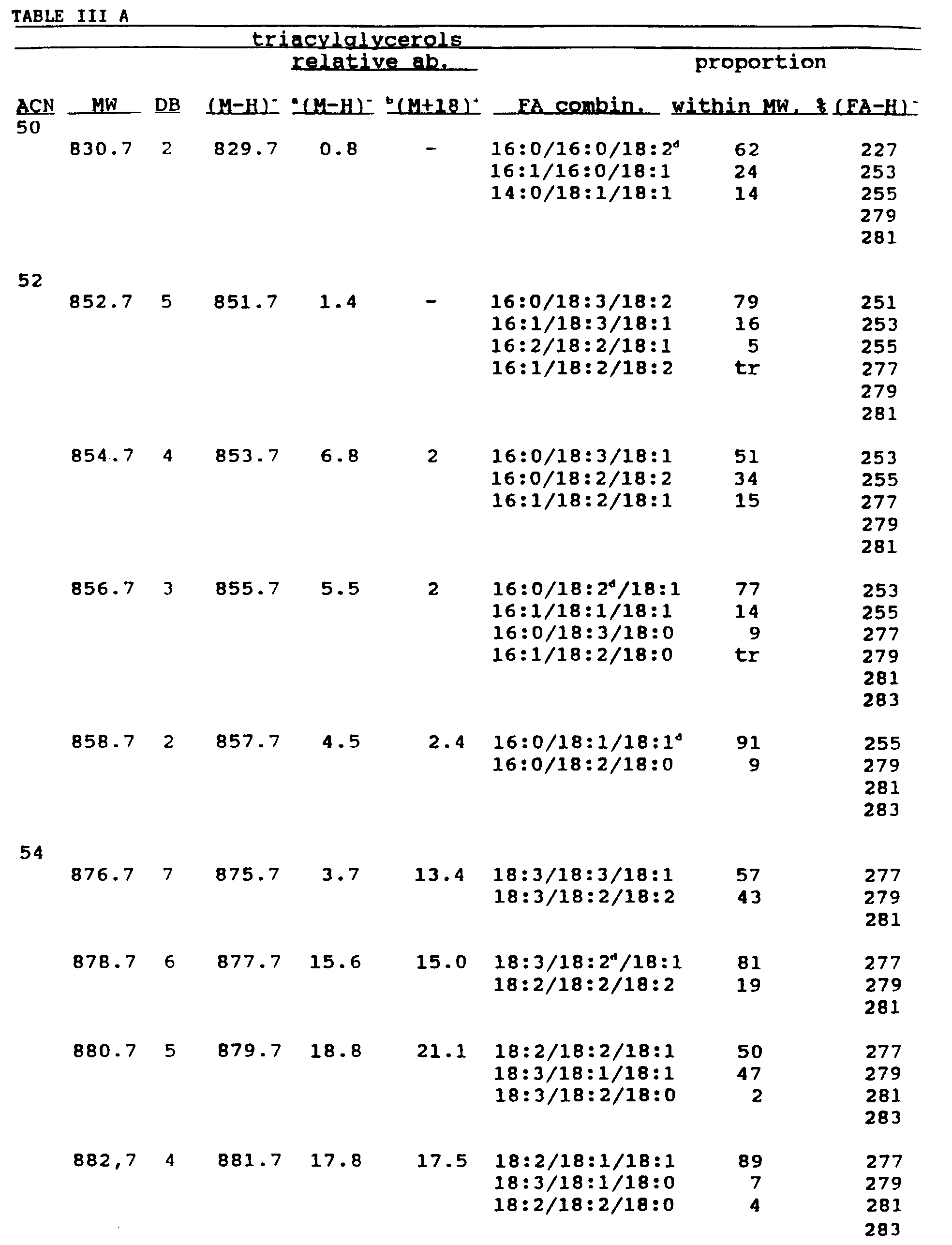

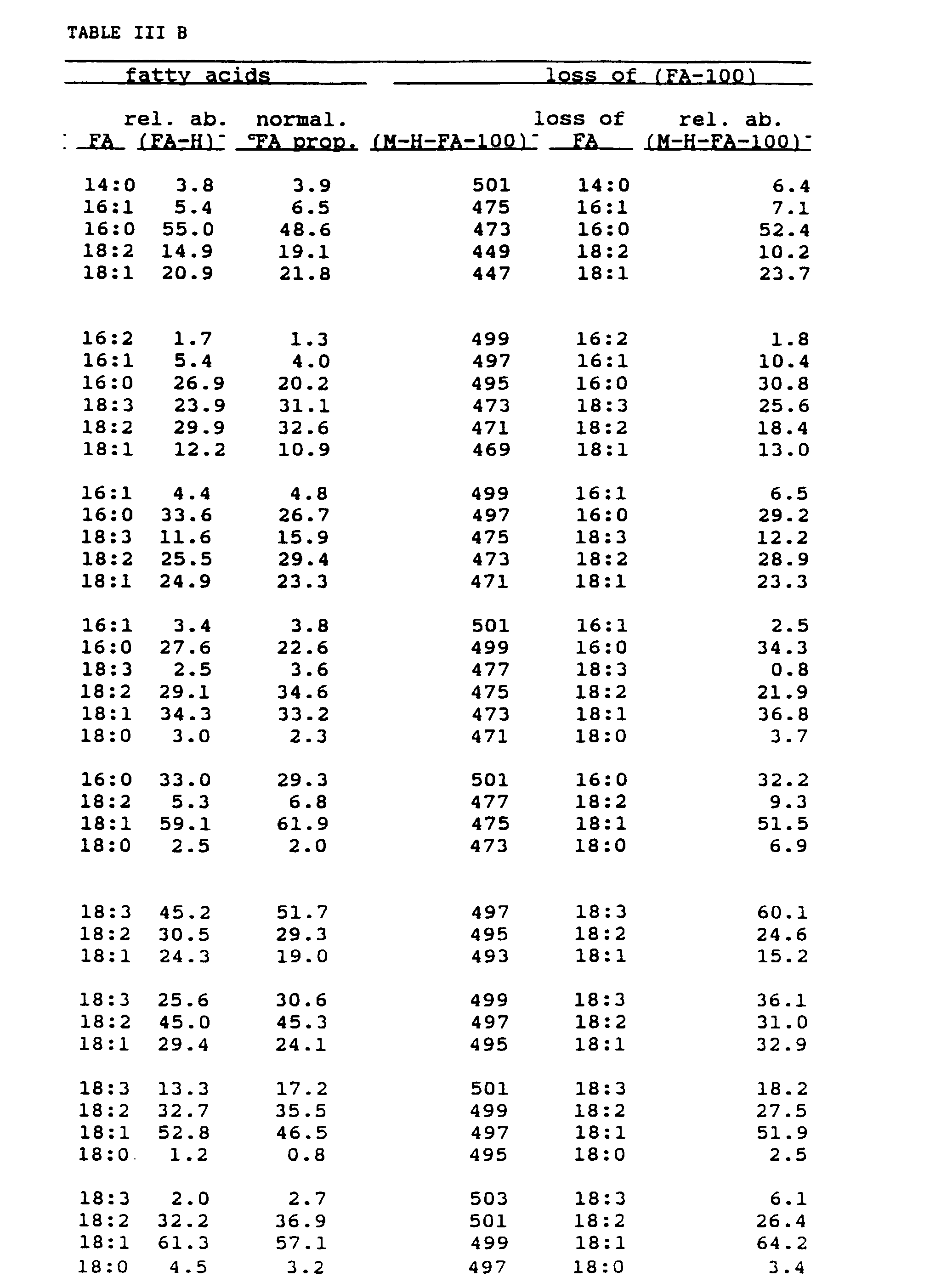

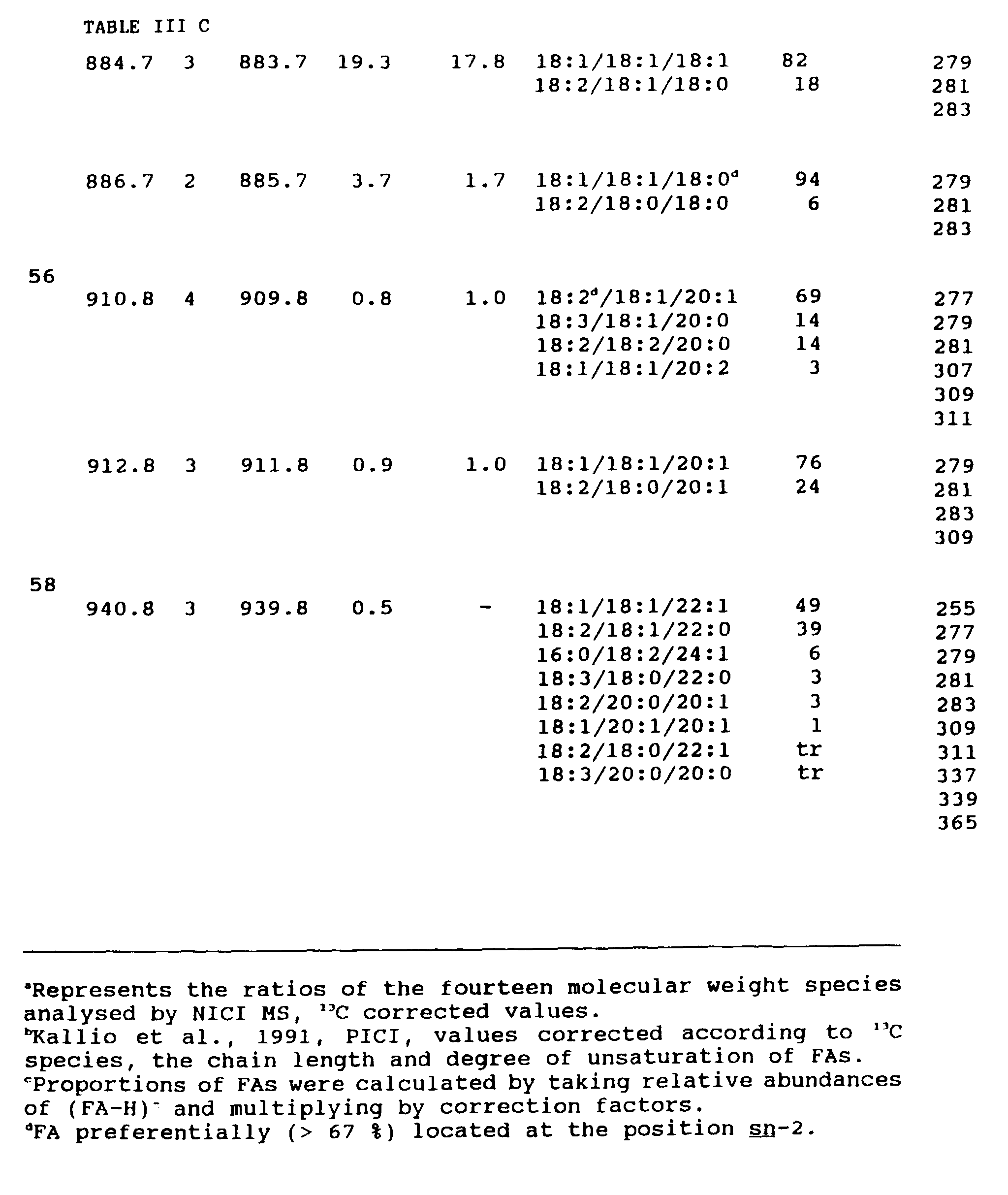

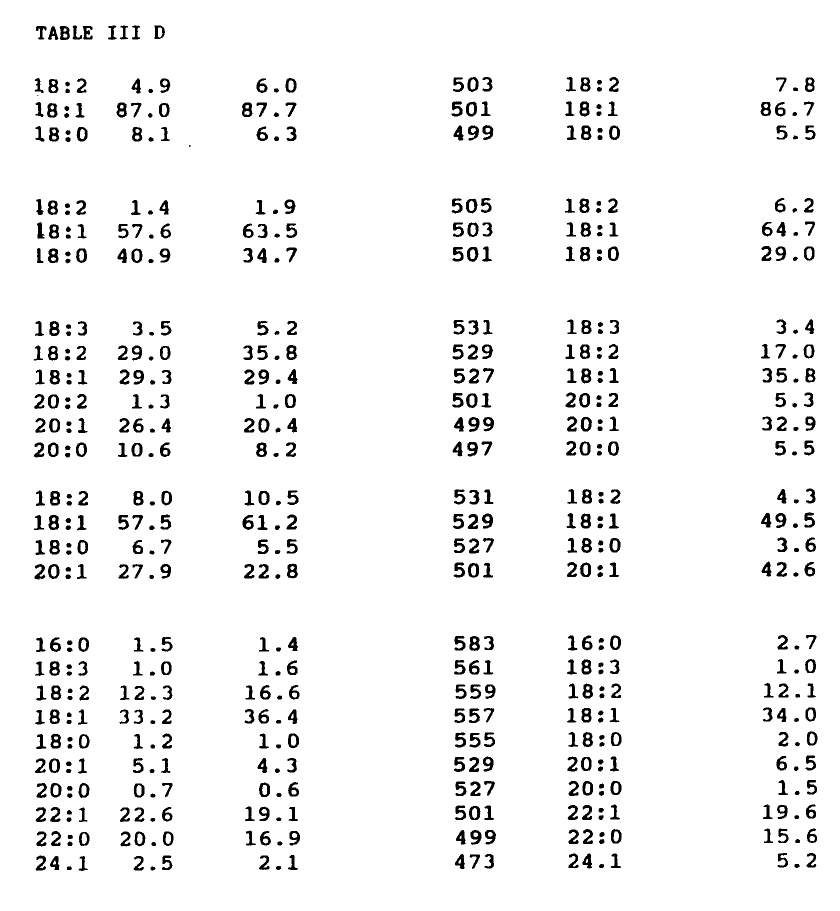

The collisional activated daughter spectra of the fourteen [M-H]⁻ ion species numbered in Figure 2 were further analyzed. The relative abundances of the RCO₂ ⁻ ions produced from the CA of the [M-H]⁻ parent TAG ion, were corrected using the correction factors listed in Table II. The corrected relative abundances of the RCO₂ ⁻ ions gave the relative proportions of fatty acids comprising that [M-H]⁻ fraction. These proportions could be used to calculate TAG structures and relative proportions of TAG mixtures, within a [M-H]⁻ ion fraction. The results are shown in Table III.

6.2. Borage oil was analyzed by ammonia negative ion chemical ionization mass spectrometry (NICI-MS). Proportions of the fatty acid combinations of TAGs of each MW species were determined from the collisional activated daughter spectra from eighteen major [M-H]⁻ species, using the corrected intensities of the RCO₂ ⁻ CA fragment ions. The resulting algebraic system was generally overdetermined and the proposed solution was computed by iteration. The results are shown in Table IV.

Table IV. Analysis of fatty acid combinations of triacylglycerols of borage oil by collisional activation of [M-H]⁻ parent ions produced by ammonia NICI of TAG mixture.

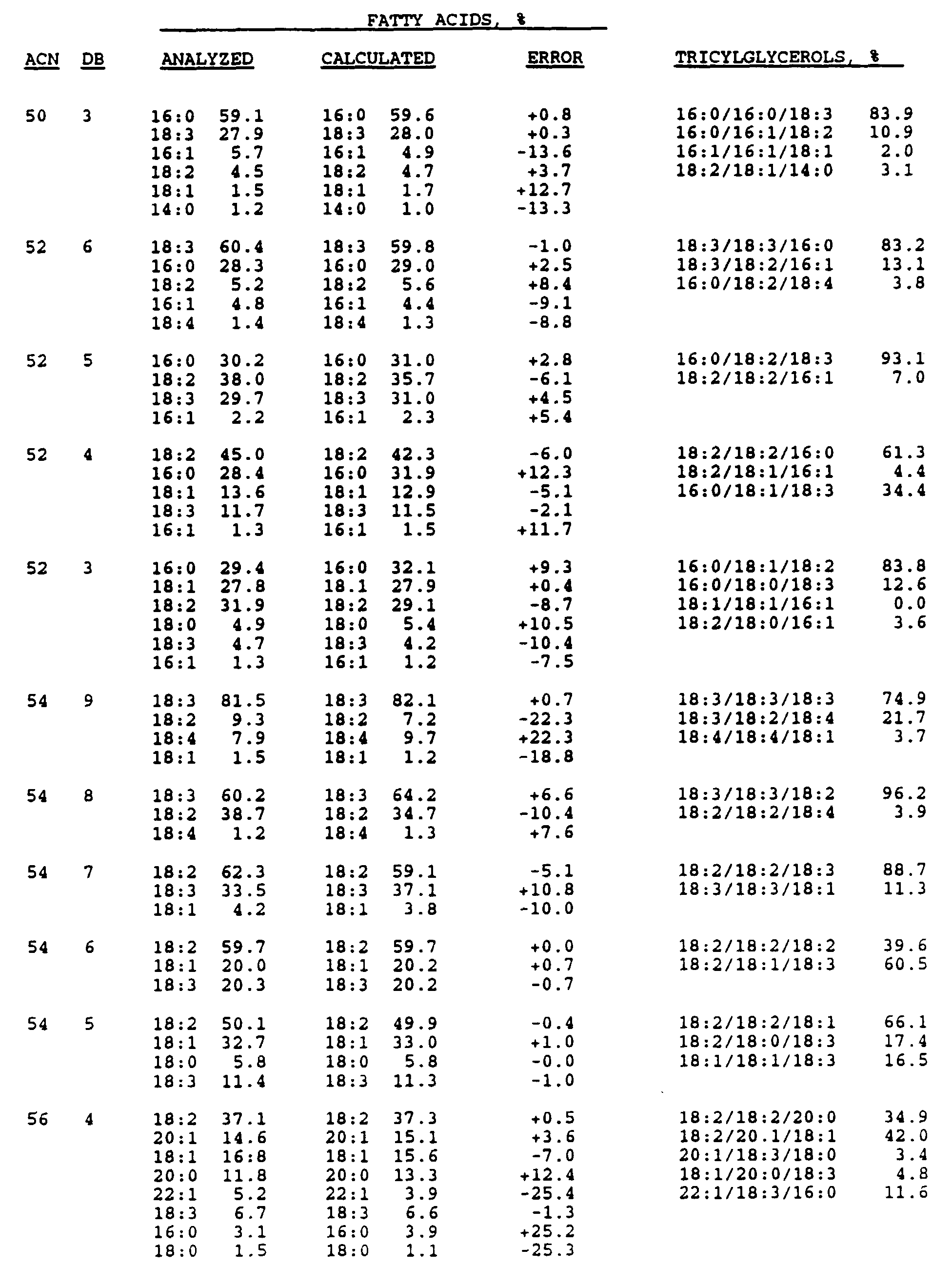

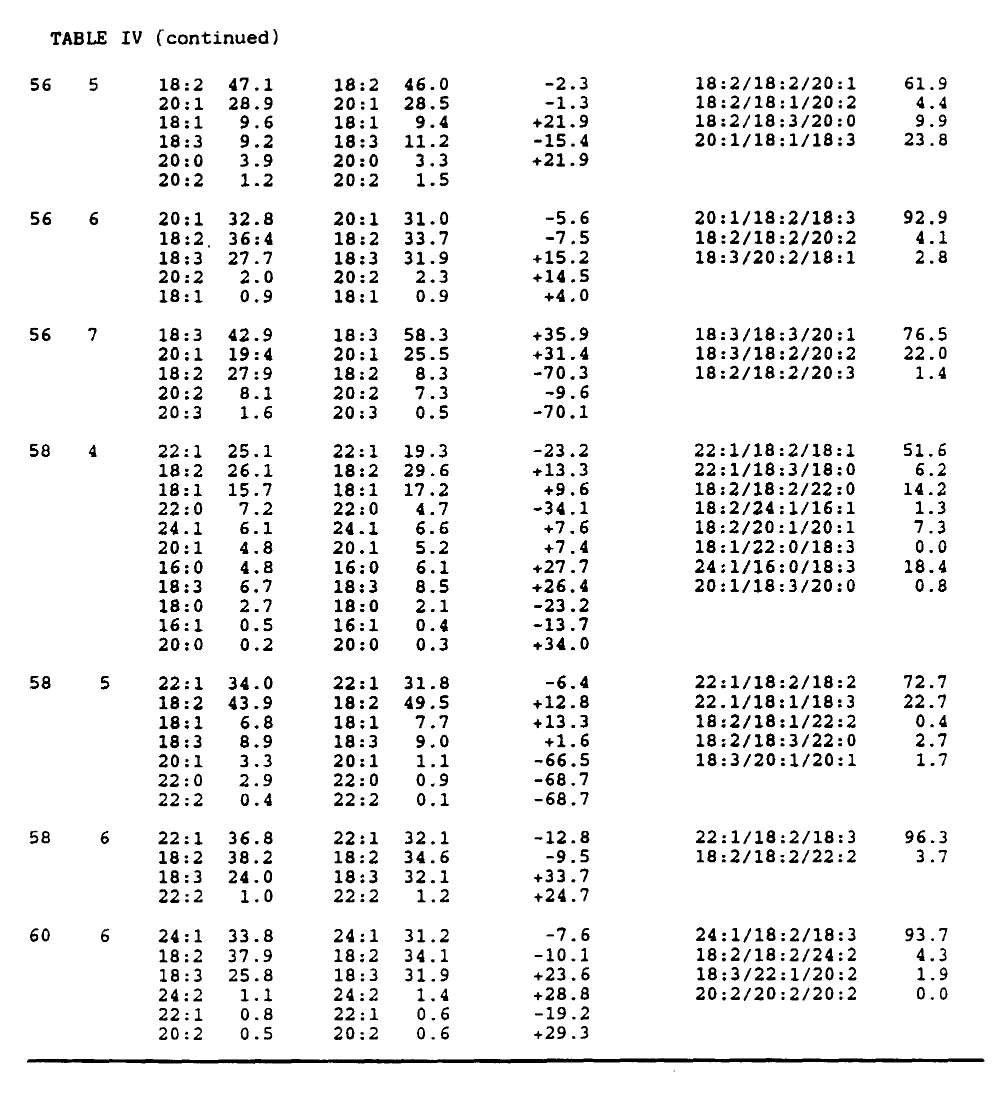

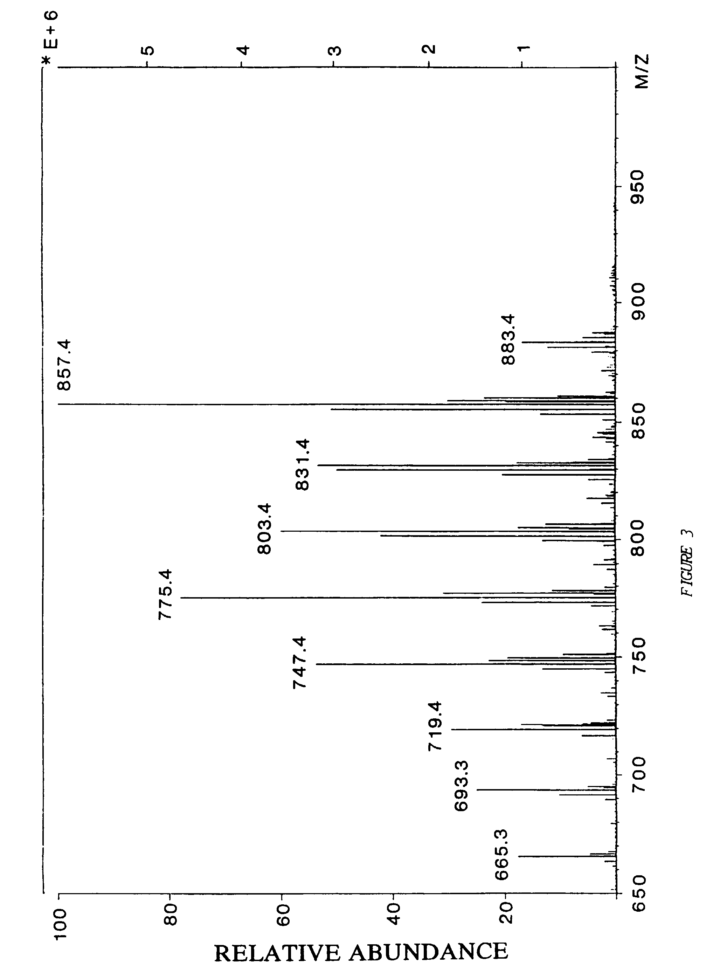

6.3. Triacylglycerols isolated from human milk were analyzed by ammonia negative ion chemical ionization mass spectrometry (NICI-MS), Figure 3. The fatty acid combinations of TAGs of 29 major MW species were determined from the collisional activated daughter spectra by using the corrected (Table II) intensities of the RCO₂ ⁻ ions.

In highly complex TAG systems, such as human milk, within one MW TAG species a large number of fatty acid combinations exist. E.g. the MW species 762.7 (ACN 45, 1 DB) consists of at least 28 fatty acid combinations. Thus when calculating the proportions of the 28 combinations several possible solutions exist. Even though it was not always possible to define accurate proportions of all the TAG combinations, the two most abundant fatty acid combinations of triacylglycerols of each MW species could be verified and the results are listed in Table V.

**Table V.**

| Major triacylglycerols (TAGs) in each of the molecular weight species analysed by collisional activation of the deprotonated TAGs. | | | | | | |
|---|---|---|---|---|---|---|
| ACN | MW | DB | major TAG | second most abundant TAG | number of FAs^{a} | number of TAGs^{b} |
| 38 | 666.7 | 0 | 12:0/12:0/14:0 | 10:0/12:0/16:0 | 5 | 4 |
| 40 | 692.7 | 1 | 10:0/12:0/18:1 | 10:0/14:0/16:1 | 10 | 9 |
| 40 | 694.7 | 0 | 12:0/12:0/16:0^{c} | 12:0/14:0/14:0 | 5 | 4 |
| 42 | 718.7 | 2 | 12:0/12:0/18:2 | 10:0/14:0/18:2 | 13 | 17 |
| 42 | 720.7 | 1 | 12:0/12:0/18:1 | 10:0/14:0^{c}/18:1 | 8 | 7 |
| 42 | 722.7 | 0 | 12:0/14:/16:0^{c} | 12:0/12:0/18:0 | 5 | 5 |
| 44 | 746.7 | 2 | 12:0/14:0^{c}/18:2 | 10:0/16:0^{c}/18:2 | 14 | 18 |
| 44 | 748.7 | 1 | 12:0/14:0^{c}/18:1 | 10:0/16:0^{c}/18:1 | 9 | 9 |
| 44 | 750.7 | 0 | 12:0/16:0/16:0 | 12:0/14:0/18:0 | 5 | 4 |
| 45 | 762.7 | 1 | 12:0/15:0^{c}/18:1 | 12:0/16:0^{c}/17:1 | 16 | 28 |
| 46 | 774.7 | 2 | 12:0/16:0^{c}/18:2 | 12:0/16:1/18:1 | 14 | 20 |
| 46 | 776.7 | 1 | 12:0/16:0^{c}/18:1 | 14:0/14:0/18:1 | 9 | 9 |
| 46 | 778.7 | 0 | 12:0/16:0^{c}/18:0 | 14:0/16:0^{c}/16:0 | 4 | 2 |
| 47 | 790.7 | 1 | 14:0/15:0/18:1 | 12:0/17:0/18:1 | 14 | 23 |
| 48 | 800.7 | 3 | 12:0^{c}/18:2/18:1 | 14:0/16:0^{c}/18:3 | 13 | 14 |
| 48 | 802.7 | 2 | 12:0/18:1/18:1 | 14:0/16:0/18:2 | 10 | 11 |
| 48 | 804.7 | 1 | 14:0/16:0^{c}/18:1 | 12:0/18:1/18:0 | 10 | 10 |
| 48 | 806.7 | 0 | 14:0/16:0^{c}/18:0 | 16:0^{c}/16:0/16:0 | 5 | 5 |
| 50 | 828.7 | 3 | 14:0^{c}/18:2/18:1 | 16:1/16:0^{c}/18:2 | 13 | 15 |
| 50 | 830.7 | 2 | 14:0^{c}/18:1/18:1 | 16:0^{c}/16:0/18:2 | 10 | 12 |
| 50 | 832.7 | 1 | 16:0^{c}/16:0/18:1 | 14:0^{c}/18:1/18:0 | 9 | 7 |
| 52 | 854.7 | 4 | 16:0^{c}/18:2/18:2 | 16:0^{c}/18:3/18:1 | 10 | 9 |
| 52 | 856.7 | 3 | 16:0^{c}/18:2/18:1 | 16:1/18:2/18:0 | 9 | 8 |
| 52 | 858.7 | 2 | 16:0^{c}/18:1/18:1 | 16:0^{c}/18:2/18:0 | 8 | 7 |
| 52 | 860.7 | 1 | 16:0^{c}/18:1/18:0 | 14:0/18:1/20:0 | 5 | 2 |
| 54 | 880.7 | 5 | 18:2/18:2/18:1 | 16:0^{c}/18:1/20:4 | 7 | 5 |
| 54 | 882.7 | 4 | 18:2/18;1/18:1 | 16:0/18:3/20:1 | 5 | 2 |
| 54 | 884.7 | 3 | 18:1/18:1/18:1 | 18:2/18:1/18:0 | 8 | 7 |
| 54 | 886.7 | 2 | 18:1/18:1/18:0 | 16:0/18:1/20:1 | 7 | 4 |

| | | | | | | |
|---|---|---|---|---|---|---|
| ^{a}Identification based on both ions m/z RCO₂ ⁻ and [M-H-RCO₂H-100]⁻ | | | | | | |
| ^{b}The number of FA combinations in TAGs, does not include the different intramolecular position isomers | | | | | | |
| ^{c}Preferentially at the position sn-2. | | | | | | |

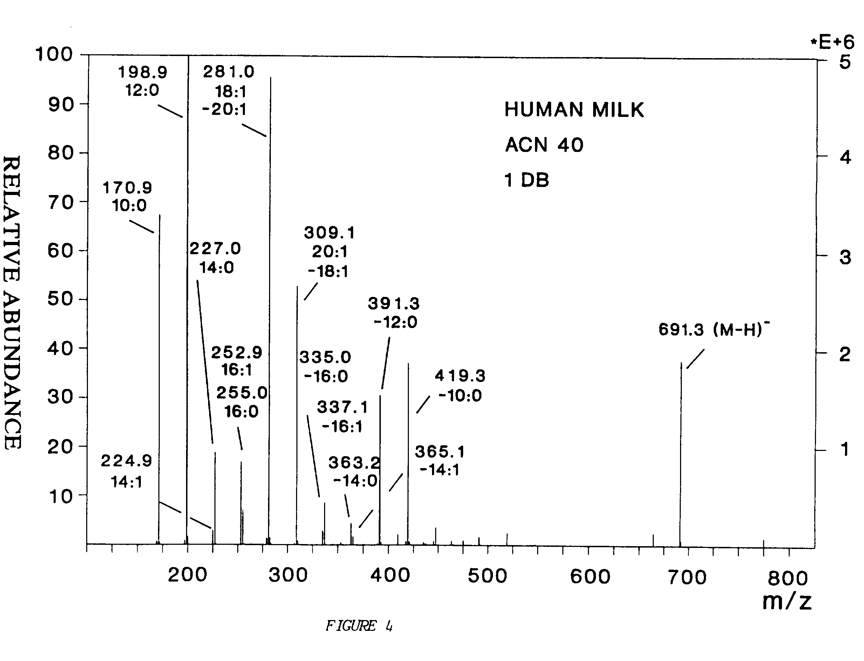

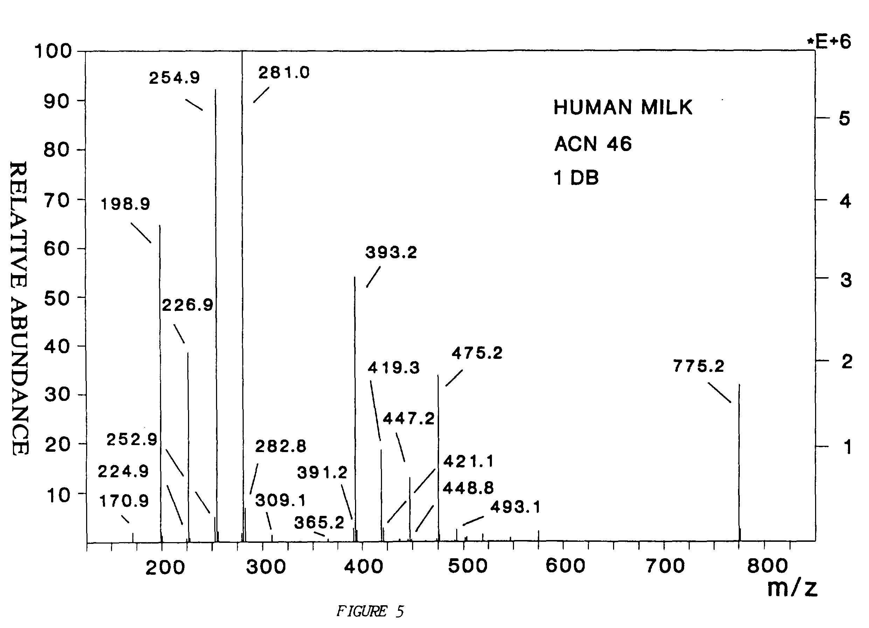

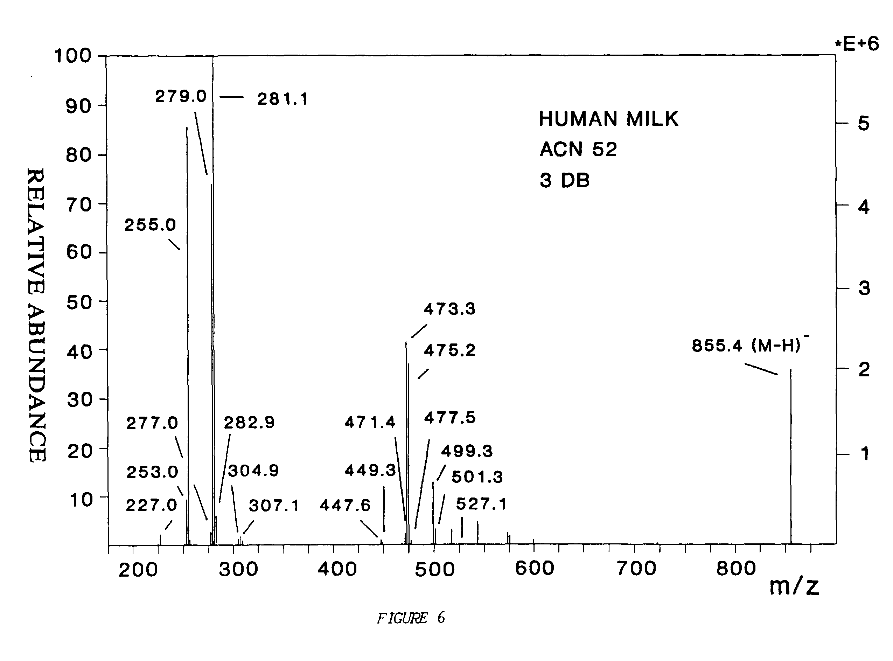

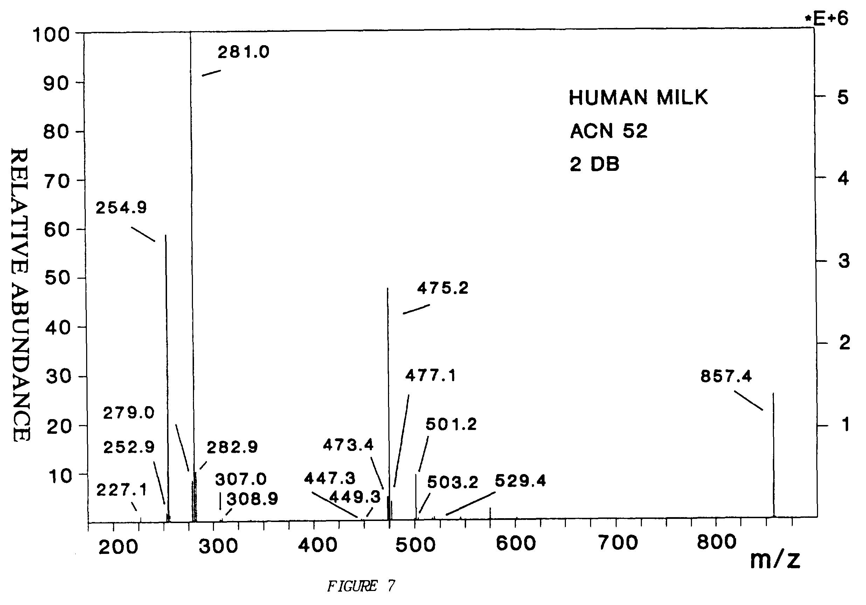

Figures 4-7 show examples of CA mass spectra of [M-H]⁻ parent ions of human milk, m/z 691.3, m/z 775.2, m/z 855.4 and m/z 857.4, respectively.

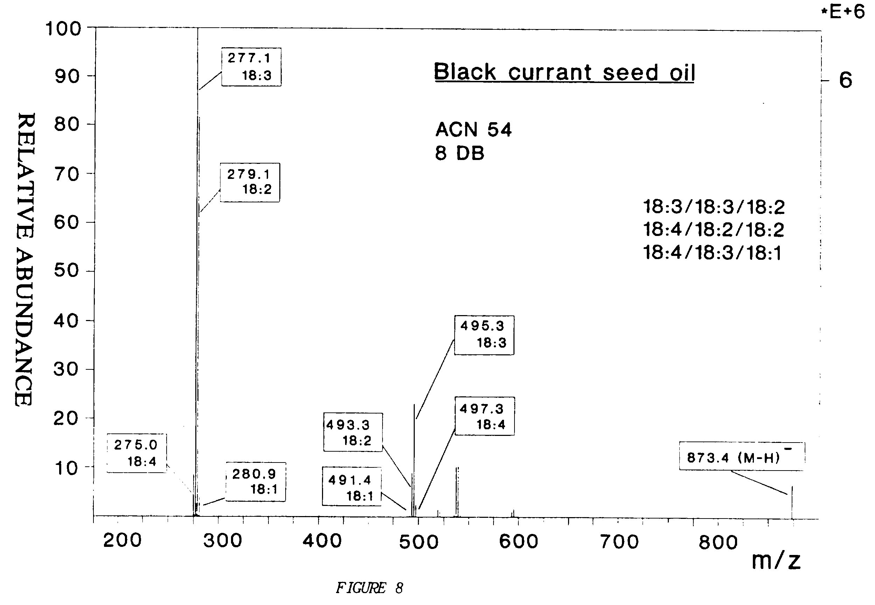

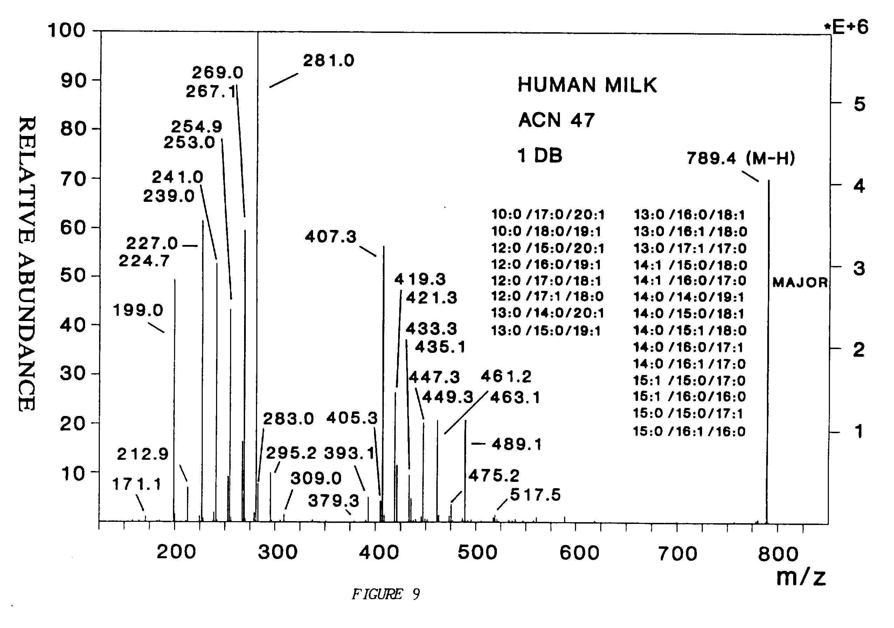

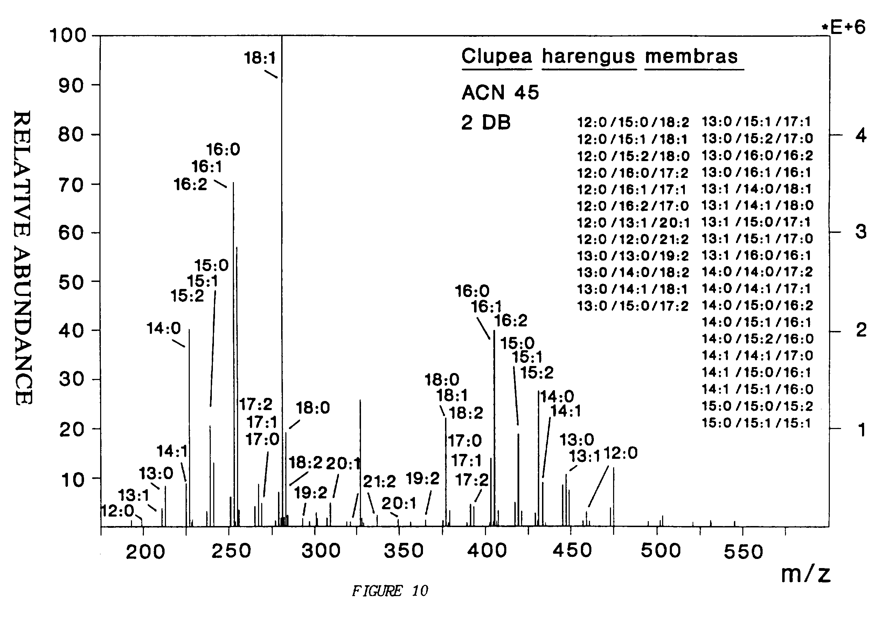

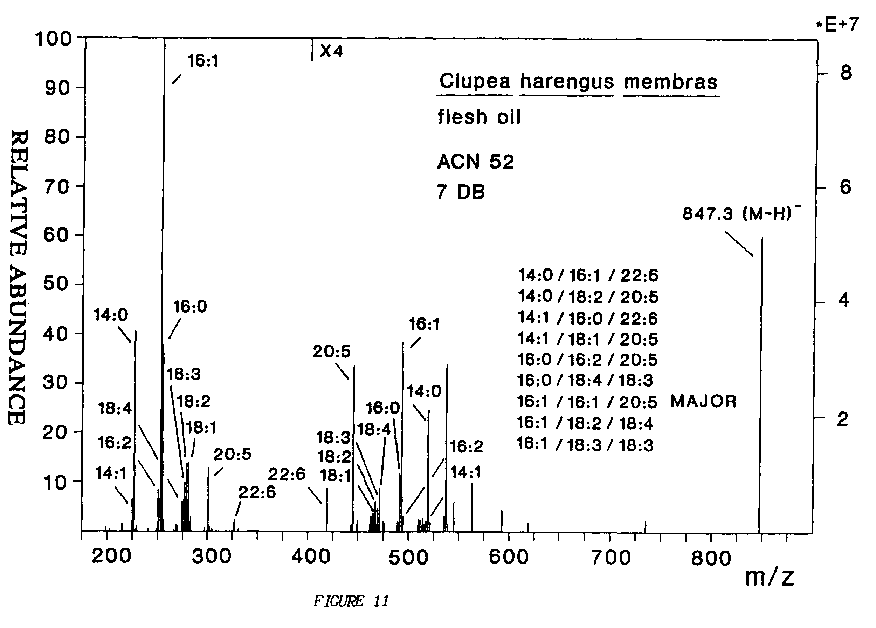

6.4. Figures 8-11 show examples of CA daughter spectra and the fatty acid combinations of TAGs of parent ion [M-H]⁻ 873.4 of black currant seed oil (Figure 8), of parent ion [M-H]⁻ 789.4 of human milk lipids (Figure 9), of parent ion [M-H]⁻ 759.4 of Baltic herring (Clupea harengus membras) flesh oil (Figure 10) and of parent ion [M-H]⁻ 847.3.4 of Baltic herring (Clupeas harengus membras) flesh oil (Figure 11).

### Example 7. Determination of the stereospecific positions of fatty acids in triacylglycerols

Both the ions RCO₂ ⁻ and [M-H-RCO₂H-100]⁻ in the CA daughter spectrum are needed to discriminate between the stereospecific positions of the fatty acyl groups in TAGs. The latter m/z area, the ions [M-H-RCO₂H-100]⁻, carries information on the molecular weights of the fatty acids like the RCO₂ ⁻ cluster does. In addition to this, the [M-H-RCO₂H-100]⁻ include information on the distribution of the fatty acids between the primary (sn-1/3) and secondary (sn-2) positions. It is not possible to distinguish between the fatty acyl groups of the two primary positions, sn-1 and sn-3, with the MS/MS method.

The intensity of [M-H-RCO₂H-100]⁻ ion is reduced when the corresponding fatty acid is esterified at the position sn-2. This discrimination is also affected by the construction of the sample introduction device and ion source to some extent. Slightly varying results with different mass spectrometers may be brought about.

As an example, partial CA-mass spectrum of the TAGs of MW 874.7 (ACN 54, 8 DB) of black currant seed oil is shown in Figure 8 and Table VI.

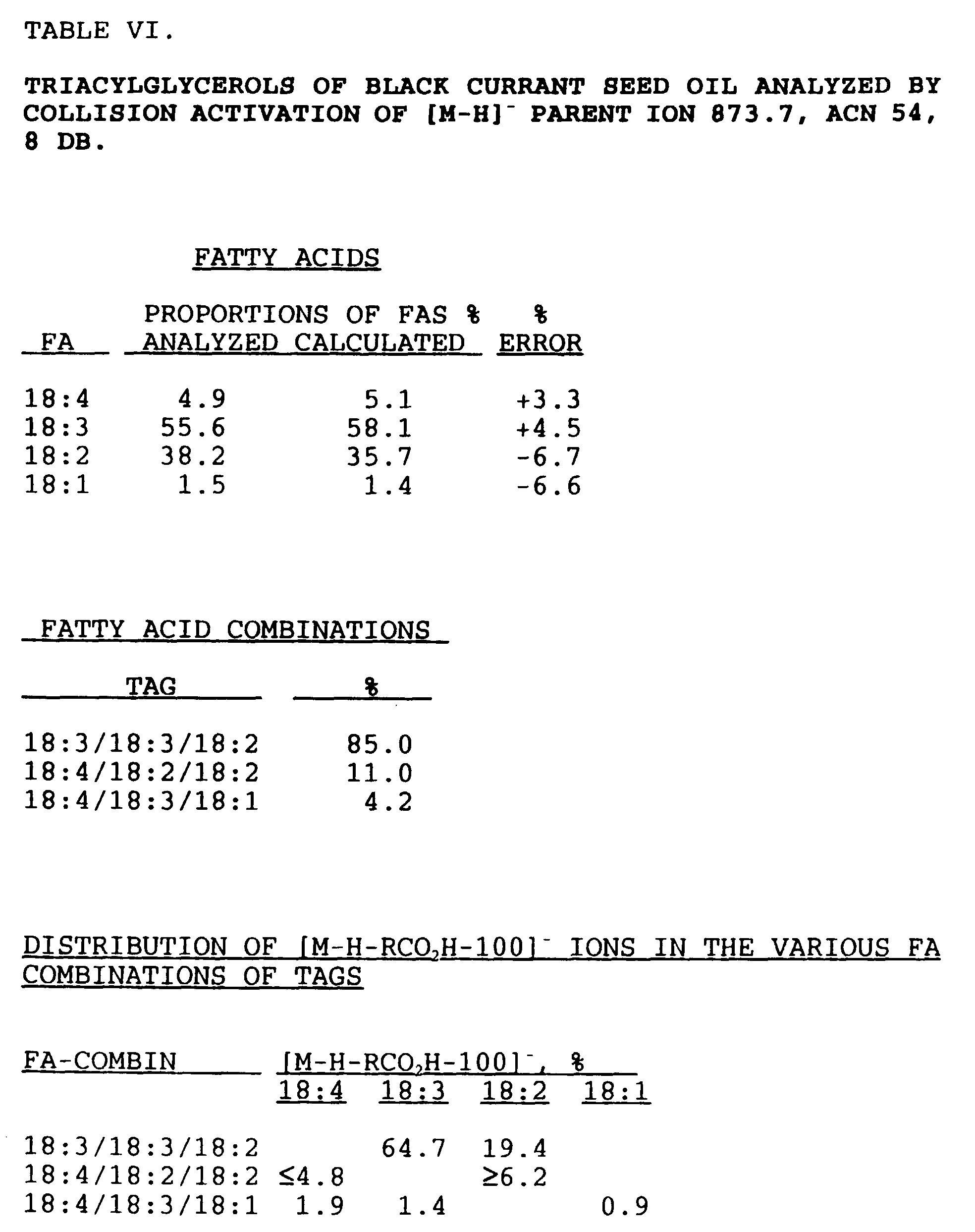

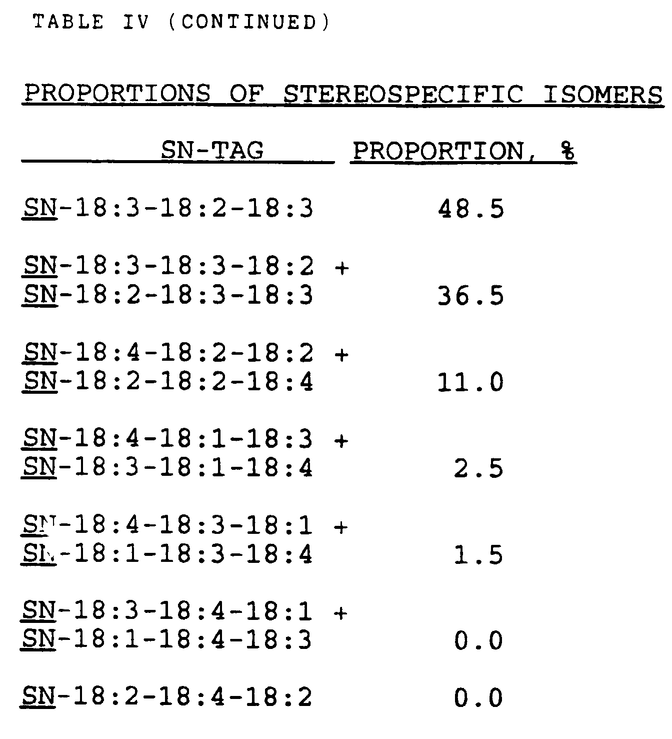

The mixture of TAGs consists of three combinations of fatty acids, i.e. 18:3/18:3/18:2 (85.0 %), 18:4/18:2/18:2 (11.0 %), and 18:4/18:3/18:1 (4.2 %) calculated from the abundances of the response-corrected RCO₂ ⁻ ions of the CA daughter spectra. The combination 18:3/18:3/18:2 is the second most abundant TAG containing the important gamma-linolenic acid and is thus one of the interesting TAGs in black currant seed oil. Proportion of the ion m/z 493.3 corresponding to [M-H-FA_{18:2}-100]⁻ is low (25.6 %) amongst the [M-H-RCO₂H-100]⁻ ions when compared with the relative intensity of the ion RCO₂ ⁻, m/z 279.1 (38.2 %) amongst the RCO₂ ⁻ ions. This shows, that the 18:2 fatty acid has at least some preference to the position sn-2 in the TAGs of MW 874.7.

The MS data of Table VI may be used to interpret the distribution of the fatty acids between the stereospecific positions.

The ion m/z 491.4 corresponding to fatty acid 18:1 and comprising 0.9 % of the [M-H-RCO₂H-100]⁻ ion cluster, originates entirely from the TAG 18:4/18:3/18:1. Because the total proportion of this TAG is 4.2 % of all the TAGs of MW 874.7, the sum of the proportions of m/z 497.3 and 495.3 derived from 18:4/18:3/18:1 is 3.3 %. According to the empirical curve of Figure 1, 63 % of FA 18:1 is at the position sn-2. Thus, only 37 % of the other fatty acids, 18:4 and 18:3, may occupy the position sn-2. Again, the calibration curve of Fig 1 gives the result, that the proportions of both of the ions m/z 497.3 (corresponding to FA 18:4) and m/z 495.3 (corresponding to FA 18:3) have to be between 1.4 and 1.9 %.

Calculations concerning ratios of stereoisomers of TAG 18:4/18:2/18:2 are based on the fact that the total proportion of the TAG as well as sum of the ions derived from it in the column [M-H-RCO₂H-100]⁻ is 11.0 %. Thus the proportion of the ion [M-H-FA_{18:2}-100]⁻ ≥6.2 %, corresponding to the end point of the calibration curve and corresponding to the TAG sn-18:2-18:4-18:2 being zero ([M-H-FA_{18:4}-100]⁻/[M-H-FA_{18:2}-100]⁻ = 0.77 and [M-H-FA_{18:4}-100]⁻ + [M-H-FA_{18:2}-100]⁻ = 11.0). This gives the amount of [M-H-FA_{18:4}-100]⁻ originating from TAGs 18:4/18:2/18:2 to be ≤4.8 %.

The total proportion of the intensity of the ion m/z 497.3 was 7.7 % according to the CA-spectrum and the closest possible resolution is, that 1.9 % of the ion originates from 18:4/18:3/18:1 and 4.8 % from 18:4/18:2/18:2. Thus 1.4 % of the [M-H-RCO₂H-100]⁻ ions comprise of m/z 495.3 originating from 18:4/18:3/18:1. Thus the fatty acid 18:4 in absolutely located at the primary positions sn-1 and/or sn-3 in TAG 18:4/18:2/18:2.

Finally, the proportions of the isomers of TAG 18:3/18:3/18:2 may be calculated from the previous data; m/z 495.3 (corresponding to FA 18:3) = 65.9 - 1.4 = 64.5 % and m/z 493.3 (corresponding to FA 18:2) = 25.5 - 6.2 = 19.4 %. These figures give the final resolution that 57 % of the fatty acid combination 18:3/18:3/18:2 is sn-18:3-18:2-18:3.

As the calculated proportions of the [M-H-RCO₂H-100]⁻ ions of the three TAGs in Table VI show, there is some error in the measurements. E.g. the intensity of the ions m/z 497.3 should be 7.7 % but according to the calculations in Table VI only 6.7 % (1.9 % + 4.8 %) of the ion exists.

Finally, Table VI shows the sn-TAGs and their proportions of molecular weight 874.7, ACN 54, 8 DB in black currant seed oil.

### Example 8. Determination of the stereospecific positions of fatty acids in triacylglycerols

Turnip rape (Brassica campestris) is a common oil seed plant, the new improved varieties of which, such as 'Kova', are low erucic acid types. The triacylglycerol fraction contains more than forty major triacylglycerol species, consisting of about twenty different fatty acids.

Applying the method developed, examples of typical results for some of the triacylglycerol species are:

8.1. A minor TAG fraction (less than 1 % of total triacylglycerols) of molecular weight (MW) 830.7 containing two Double Bonds (DB) and 50 Acyl Carbons (ACN 50) each, consisted of five different fatty acids, i.e. 14:0, 16:1, 16:0, 18:2 and 18:1.

The TAGs consisted of fatty acid combinations 16:0/16:0/18:2 (62 %), 16:1/16:0/18:1 (24 %) and 14:0/18:1/18:1 (14 %) (In these formulae the fatty acids are in the order of their molecular weights and do not express the stereospecific positions of the fatty acids in the TAGs). Calculations were based on the intensities of the RCO₂ ⁻ ions of the collisional activated daughter spectra of the [M-H]⁻ ions (m/z 829.7).

When taking the stereospecific positions of the fatty acyl groups into account, the TAGs consisted of sn-16:0-18:2-16:0 (50 %), mixture of sn-16:1-16:0-18:1 and sn-18:1-16:0-16:1 (24 %), mixture of sn-14:0-18:1-18:1 and sn-18:1-18:1-14:0 (14 %) and of mixture of sn-16:0-16:0-18:2 and 18:2-16:0-16:0 (12 %). The fatty acids set in the middle positions in the formulae represent the position sn-2. Calculations were based on both the intensities of the RCO₂ ⁻ ions and [M-H-RCO₂H-100]⁻ ions of the collisional activated daughter spectra of the [M-H]⁻ ions (m/z 829.7).

The method of the present invention applied herein does not distinguish the fatty acids between the positions sn-1 and sn-3 in the triacylglycerol.

8.2. A mid-size TAG fraction (5 % of total TAGs) of MW 858.7, 2 DB, ACN 52, consisted of four different fatty acids, i.e. 16:0, 18:2, 18:1 and 18:0.

The TAGs consisted of fatty acid combinations 16:0/18:1/18:1 (91 %) and 16:0/18:2/18:0 (9 %) in which formulae the fatty acids are in the order of their molecular weights and do not express the stereospecific positions of the fatty acids in the TAGs. Calculations were based on the intensities of the RCO₂ ⁻ ions of the collisional activated daughter spectra of the [M-H]⁻ ions (m/z 857.7).

When taking the stereospecific positions of the fatty acyl groups into account, the TAGs consisted of the mixture of sn-16:0-18:1-18:1 and sn-18:1-18:1-16:0 (76 %), sn-18:1-16:0-18:1 (15 %) and of the mixture of sn-18:2-16:0-18:0 and sn-18:0-16:0-18:2 (9 %). The fatty acids set in the middle positions in the formulae represent the position sn-2. Calculations were based on both the intensities of the RCO₂ ⁻ ions and [M-H-RCO₂H-100]⁻ ions of the collisional activated daughter spectra of the [M-H]⁻ ions (m/z 857.7).

The method of the present invention applied herein does not distinguish the fatty acids between the positions sn-1 and sn-3 in the triacylglycerol.

8.3. The most abundant triacylglycerol (15 %) amongst all the TAGs of turnip rape seedoil was 18:1-18:1-18:1 of MW 884.7, ACN 54 and 3 DB.

### Example 9. Determination of the stereospecific positions of fatty acids in triacylglycerols

Baltic herring (Clupea harengus membras) is a widely used North-European brackish water fish closely related to herring. Fish oils are known to consist typically of more than one hundred fatty acids, which form thousands of different triacylglycerols.

Applying the method developed, typical examples of results of some of the Baltic herring flesh oil triacylglycerol species are:
9.1. The MW 828.7 triacylglycerol species (ACN 50, 3 DB) consists of seven major fatty acids (14:0, 16:2, 16:1, 16:0, 18:3, 18:2 and 18:1) and five different fatty acid combinations in TAGs, i.e.: 16:1/16:1/18:1 (54 %), 14:0/18:2/18:1 (20 %), 16:1/16:0/18:2 (14 %), 16:0/16:0/18:3 (10 %) and 16:2/16:0/18:1 (3 %) (The formulae of the fatty acids are in the order of their molecular weights and do not express the stereospecific positions of the fatty acids in the TAGs). Calculations were based on the intensities of the RCO₂ ⁻ ions of the collisional activated daughter spectra of the [M-H]⁻ ions (m/z 827.7).

The major fatty acid combination 16:1/16:1/18:1 consisted mainly (> 90 %) of TAGs sn-16:1-16:1-18:1 and sn-18:1-16:1-16:1. Only trace amounts of TAG sn-16:1-18:1-16:1 existed. The fatty acids set in the middle positions in the formulae represent the position sn-2. Calculations were based on both the intensities of the RCO₂ ⁻ ions and [M-H-RCO₂H-100]⁻ ions of the collisional activated daughter spectra of the [M-H]⁻ ions (m/z 827.7).

9.2. The MW 832.7 triacylglycerol species (ACN 50, 1 DB) consists typically of five major fatty acids (14:0, 16:1, 16:0, 18:1 and 18:0) and three different fatty acid combinations in TAGs, i.e.: 16:0/16:0/18:1 (80 %), 16:1/16:0/18:0 (20 %) and 14:0/18:1/18:0 (trace)in which formulae the fatty acids are in the order of their molecular weights and do not express the stereospecific positions of the fatty acids in the TAGs. Calculations were based on the intensities of the RCO₂ ⁻ ions of the collisional activated daughter spectra of the [M-H]⁻ ions (m/z 831.7).

The major fatty acid combination 16:0/16:0/18:1 showed an almost random distribution of the fatty acids in the three stereospecific positions of TAGs.

The second most abundant combination 16:1-16:0-18:0 consisted entirely of TAGs sn-16:0-16:1-18:1 and sn-18:1-16:1-16:0. The fatty acids set in the middle positions in the formulae represent the position sn-2. Calculations were based on both the intensities of the RCO₂ ⁻ ions and [M-H-RCO₂H-100]⁻ ions of the collisional activated daughter spectra of the [M-H]⁻ ions (m/z 831.7).

9.3. The MW 834.7 triacylglycerol species (ACN 50, 0 DB) consists of four major fatty acids (14:0, 16:0, 18:0 and 20:0) which comprise three different fatty acid combinations in TAGs, i.e.:, 16:0/16:0/18:0 (87 %), 14:0/16:0/20:0 (9 %) and 14:0/18:0/18:0 (4 %) (the formulae of the fatty acids are in the order of their molecular weights and do not express the stereospecific positions of the fatty acids in the TAGs). Calculations were based on the intensities of the RCO₂ ⁻ ions of the collisional activated daughter spectra of the [M-H]⁻ ions (m/z 833.7).

The major fatty acid combination 16:0/16:0/18:0 consisted mainly (> 80 %) of TAGs sn-16:0-16:0-18:0 and sn-18:0-16:0-16:0. Only trace amounts of TAG sn-16:0-18:0-16:0 existed. The fatty acids set in the middle positions in the formulae represent the position sn-2. Calculations were based on both the intensities of the RCO₂ ⁻ ions and [M-H-RCO₂H-100]⁻ ions of the collisional activated daughter spectra of the [M-H]⁻ ions (m/z 833.7).

9.4. The MW 856.7 triacylglycerol species (ACN 52, 3 DB) consists of eight major fatty acids (14:0, 16:1, 16:0, 18:3, 18:2, 18:1, 18:0 and 20:3) which comprise six different fatty acid combinations in TAGs, i.e.: 16:1/18:1/18:1 (50 %), 16:0/18:2/18:1 (47 %), 16:0/18:3/18:0 (3 %), 14:0/18:0/20:3 (1 %), 16:0/16:0/20:3 (trace) and 16:1-18:2-18:0 (trace) (the formulae the fatty acids are in the order of their molecular weights and do not express the stereospecific positions of the fatty acids in the TAGs). Calculations were based on the intensities of the RCO₂ ⁻ ions of the collisional activated daughter spectra of the [M-H]⁻ ions (m/z 855.7).

Of the major fatty acid combination 16:1/18:1/18:1 the TAG sn-18:1-16:1-18:1 comprised about half of the positional isomers. The fatty acids set in the middle positions in the latter formula represent the position sn-2. Calculations were based on both the intensities of the RCO₂ ⁻ ions and [M-H-RCO₂H-100]⁻ ions of the collisional activated daughter spectra of the [M-H]⁻ ions (m/z 855.7).

9.5. The MW 858.7 triacylglycerol species (ACN 52, 2 DB) consists of seven major fatty acids (14:0, 16:1, 16:0, 18:2, 18:1, 18:0 and 20:1) which comprise five different fatty acid combinations in TAGs, i.e.: 16:0/18:1/18:1 (85 %), 16:1/18:1/18:0 (11 %), 14:0/18:1/20:1 (3 %), 16:1/16:0/20:1 (1 %) and 16:0/18:2/18:0 (trace) (the formulae the fatty acids are in the order of their molecular weights and do not express the stereospecific positions of the fatty acids in the TAGs). Calculations were based on the intensities of the RCO₂ ⁻ ions of the collisional activated daughter spectra of the [M-H]⁻ ions (m/z 857.7).

Of the major fatty acid combination 16:0/18:1/18:1 the TAG sn-18:1-16:0-18:1 comprised about half of the positional isomers. The fatty acids set in the middle positions in the latter formula represent the position sn-2. Calculations were based on both the intensities of the RCO₂ ⁻ ions and [M-H-RCO₂H-100]⁻ ions of the collisional activated daughter spectra of the [M-H]⁻ ions (m/z 857.7).

### Example 10. Determination of the stereospecific positions of fatty acids in triacylglycerols

In highly complex TAG systems, such as human milk, within one MW TAG species a large number of fatty acid combinations exist (Table V). The accurate determination of the stereospecific positions of the fatty acyl groups in the TAGs is thus not always possible. However, in most cases the possible non-random distribution of the fatty acids in the stereospecific positions could be determined, i.e. preferences for the sn-2 position could be observed. The results are shown in Table V.

### Example 11. Determination of the stereospecific positions of fatty acids in triacylglycerols

It is common knowledge, that in many seed oils linoleic acid (18:2, hexadecadienoic acid) is generally preferentially located at the position sn-2. The proportion of 18:2 fatty acid locating at the position sn-2 was analyzed of triacylglycerols of ACN 52/ 5 DB, ACN 54/ 7 DB and ACN 54/ 8 DB of borage oil, black currant seed oil, low erucic acid turnip rapeseed oil and three samples of evening primrose oil. These three combinations constitute about 3/4 of the TAGs of evening primrose oil and about 1/2 of borage oil and black currant oil. In turnip rape, these TAG species exist in trace amount only.

11.1. Triacylglycerols 16:0/18:3/18:2 of molecular weight 852.7 (ACN 52/ 5 DB) contained TAGs 16:0-18:2-18:3 (sum of sn-16:0-18:2-18:3 and sn-18:3-18:2-16:0) in each oil as follows (proportion of 16:0/18:3/18:2 of the TAGs in oil in parentheses):

| | |
|---|---|
| evening primrose I | 50-60 % (14 %) |
| evening primrose II | 50-55 % (20 %) |
| evening primrose III | 45 % (14 %) |
| borage | 30 % (15 %) |
| black currant | 60-65 % (14 %) |
| turnip rape | 50-65 % ( 1 %) |

11.2. Triacylglycerols 18:3/18:3/18:2 of molecular weight 874.7 (ACN 54/ 8 DB) contained TAG 18:3-18:2-18:3 in each oil as follows (proportion of 18:3/18:3/18:2 of the TAGs in oil in parentheses):

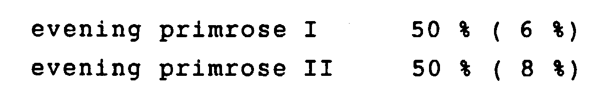

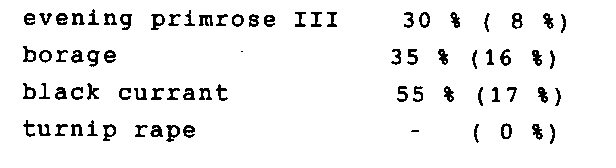

11.3. Triacylglycerols 18:3/18:2/18:2 of molecular weight 876.7 (ACN 54/ 7 DB) contained TAGs 18:3-18:2-18:2 (sum of sn-18:3-18:2-18:2 and sn-18:2-18:2-18:3) in each oil as follows (proportion of 18:3/18:2/18:2 of the TAGs in oil in parentheses):

| | |
|---|---|
| evening primrose I | 100 % (53 %) |
| evening primrose II | 85 % (51 %) |
| evening primrose III | 85 % (55 %) |
| borage | 65 % (20 %) |
| black currant | 90- 95 % (26 %) |
| turnip rape | 100 % ( 2 %) |

## Claims

1. A method of determination of structural information of a triacylglycerol or a mixture of triacylglycerols comprising the steps of;
a) generating within a mass spectrometer molecular or quasi-molecular ions which retain the substantial structure of the triacylglycerol or triacylglycerols,
subjecting the ions to a first mass analyser selection process to resolve the ions into first fractions of a particular mass/charge ratio and
determining the relative abundance of each first fraction and
selecting in turn each of the first fractions,
b) collisionally activating the ions within each selected first fraction and
allowing them or forcing them to decompose into fragment ions,
subjecting the fragment ions to a second mass analyser selection process to resolve the ions into fragments of a particular mass/charge ratio and
determining the relative abundance of each fragment ion,
c) selecting from the ions collisionally activated in step b) the following
RCO₂ ⁻ to show the fatty acids (RCO₂H) and their proportions of the selected parent ion triacylglycerols and
[M-H-RCO₂-X]⁻, wherein X is a constant mass, for the different fatty acids to show the distribution of the same between the primary (sn-1/3) and secondary (sn-2) positions in the triacylglyserols so that the proportion [M-H-B-X]⁻ to [M-H-A-X]⁻, wherein, A and B are different fatty acids, is compared to a constant curve received empirically using known fatty acids.

2. A method according to claim 1, wherein X in step c) is 100.

3. A method according to claim 1, wherein the proportion [M-H-B-X]⁻ to [M-H-A-X]⁻ is between about 0,145 and about 0,784.

4. A method according to claim 1 in which the molecular or quasi-molecular ions are generated by positive or negative ion chemical ionisation procedures.

5. A method according to claim 4 in which the molecular or quasi-molecular ions are generated by negative ion chemical ionisation procedures.

6. A method according to claim 5 in which the molecular or quasi-molecular ions are generated by ammonia negative ion chemical ionisation procedures.

7. A method according to claim 1, in which it is used a mass spectrometer that is an ion trap or ion cyclotron resonance mass spectrometer using sequential mass analysing in time.

8. A method according to claim 1, in which it is used a mass spectrometer that is one using metastable scanning, e.g. linked scans.

## Patentansprüche

1. Verfahren zur Bestimmung der Strukturinformation eines Triacylglycerols oder einer Mischung von Triacylglycerolen, umfassen die Schritte:
a) Erzeugen von Molekül- oder Quasi-Molekülionen, welche die wesentliche Struktur des Triacylglycerols oder der Triacylglycerole beibehalten, innerhalb eines Massenspektrometers.
Unterziehen der Ionen einem ersten Massenanalysator-Selektionsverfahren, um die Ionen in erste Fraktionen mit einem bestimmten Masse/Ladungs-Verhältnis aufzulösen und
Bestimmen der relativen Häufigkeit jeder ersten Fraktion und
der Reihe nach Auswählen jeder der ersten Fraktionen.
b) Stoßaktivieren der Ionen innerhalb jeder ausgewählten ersten Fraktion und
Zersetzenlassen oder Bewirken einer Zersetzung dieser zu Fragmentionen,
Unterziehen der Fragmentionen einem zweiten Massenanalysator-Selektionsverfahren, um die Ionen in Fragmente mit einem bestimmten Masse/Ladungs-Verhältnis aufzulösen, und
Bestimmen der relativen Häufigkeit jedes Fragmentions,
c) Auswählen des folgenden aus den in Schritt b) stoßaktivierten Ionen RCO₂ ⁻, um die Fettsäuren (RCO₂H) und deren Anteile in den ausgewählten Stammtriacylglycerolionen zu zeigen, und
[M-H-RCO₂-X)⁻, worin X eine konstante Masse ist, für die verschiedenen Fettsäuren, um deren Verteilung zwischen der primären (sn-1/3) und sekundären (sn-2) Position in den Triacylglycerolen zu zeigen, so daß das Verhältnis [M-H-B-X]⁻ zu [M-H-A-X]⁻, worin A und B verschiedene Fettsäuren sind, mit einer konstanten Kurve, welche empirisch unter Verwendung bekannter Fettsäuren erhalten worden ist, verglichen wird.

2. Verfahren nach Anspruch 1, wobei X beim Schritt c) 100 ist.

3. Verfahren nach Anspruch 1, wobei das Verhältnis [M-H-B-X]⁻ zu [M-H-A-X]⁻ zwischen etwa 0,145 und etwa 0,784 liegt.

4. Verfahren nach Anspruch 1, wobei die Molekül- oder Quasi-Molekülionen durch chemische Ionisationsverfahren unter Verwendung positiver oder negativer Ionen erzeugt werden.

5. Verfahren nach Anspruch 4, wobei die Molekül- oder Quasi-Molekülionen durch chemische Ionisationsverfahren unter Verwendung negativer Ionen erzeugt werden.

6. Verfahren nach Anspruch 5, wobei die Molekül- oder Quasi-Molekülionen durch chemische Ionisationsverfahren unter Verwendung negativer Ammoniakionen erzeugt werden.

7. Verfahren nach Anspruch 1, bei dem ein Massenspektrometer eingesetzt wird, das ein Ionenfallen- oder Ionenzyklotron-Resonanzmassenspektrometer ist, bei dem eine zeitabhängige sequentielle Massenanalyse durchgeführt wird.

8. Verfahren nach Anspruch 1, bei dem ein Massenspektrometer eingesetzt wird, das unter Anwendung metastabilen Scannings, beispielsweise gekoppelten Ablenkungen, arbeitet.

## Revendications

1. Méthode de détermination d'informations sur la structure d'un triacylglycérol ou d'un mélange de triacylglycérols comprenant les étapes :
(a) de génération au sein d'un spectromètre de masse, d'ions moléculaires ou quasi-moléculaires qui conservent la structure essentielle du triacylglycérol ou des triacylglycérols,
en soumettant les ions à une première procédure de sélection sur analyseur de masse de façon à séparer les ions en premières fractions d'un ratio particulier masse particulaire/charge, et
en déterminant l'abondance relative de chaque première fraction, et
en sélectionnant à leur tour chacune des premières fractions,
(b) d'activation des ions par collision, au sein de chaque première fraction sélectionnée,
en leur permettant de, ou en les forçant à se décomposer en ions fragmentés,
en soumettant les ions fragmentés à une seconde procédure de sélection sur analyseur de masse de façon à séparer les ions en fragments d'un ratio particulier masse particulaire/charge, et
en déterminant l'abondance relative de chaque ion fragmenté,
(c) de sélection à partir des ions activés par collision à l'étape (b), du RCOO⁻ suivant, pour indiquer les acides gras (RCOOH) et leurs proportions en ions parents triacylglycérols et [M-H-RCOO-X]⁻, où X est une masse constante, pour les différents acides gras, pour montrer la distribution de ceux-ci entre les positions primaires (sn-1/3) et secondaires (sn-2) dans les triacylglycérols, de façon à ce que la proportion [M-H-B-X]⁻/[M-H-A-X]⁻ dans laquelle A et B sont des acides gras différents, soit comparée à une courbe constante obtenue empiriquement à l'aide d'acides gras connus.

2. Méthode selon la revendication 1, dans laquelle X à l'étape (c), est égal à 100.

3. Méthode selon la revendication 1, dans laquelle la proportion [M-H-B-X]⁻/[M-H-A-X]⁻ est comprise entre environ 0,145 et environ 0,784.

4. Méthode selon la revendication 1, dans laquelle les ions moléculaires ou quasi-moléculaires sont générés par des procédures d'ionisation chimique en ions positifs ou négatifs.

5. Méthode selon la revendication 4, dans laquelle les ions moléculaires ou quasi-moléculaires sont générés par des procédures d'ionisation chimique en ions négatifs.

6. Méthode selon la revendication 5, dans laquelle les ions moléculaires ou quasi-moléculaires sont générés par des procédures d'ionisation chimique à l'ammoniac, en ions négatifs.

7. Méthode selon la revendication 1, dans laquelle on emploie un spectromètre de masse qui est un spectromètre de masse à résonance de piège à ion ou de cyclotron à ion, en utilisant une analyse séquentielle de masse en fonction du temps.

8. Méthode selon la revendication 1, dans laquelle on emploie un spectromètre de masse utilisant le balayage métastable, par exemple, des balayages liés.
